# EUROPEAN PATENT APPLICATION

(11) **EP 4 840 909 A1**
(43) Date of publication of application: **18.11.2026**
(21) Application number: 25176403.1
(22) Date of filing: 14.05.2025
(51) Int. Cl.: C09B 11/10, C09B 11/24, C09B 11/28, C09B 19/00, C09B 21/00, C09B 56/00, C12Q 1/00, G01N 33/50

(54) **AMINE-SUBSTITUTED POLYCYCLIC FLUORESCENCE QUENCHER DYES**

(71) Applicant: ATTO-TEC Gmbh, 57074 Siegen (DE)
(72) Inventor: ZILLES, Alexander, 57074 Siegen (DE); WICKHORST, Peter, 57074 Siegen (DE)
(74) Representative: Maiwald GmbH

(57) **Abstract**

The present invention relates to polycyclic compounds of general formula (I), to processes of their preparation, and to the use thereof as fluorescence quenchers in energy-transfer assays and applications. The invention further relates to bi-chromophoric systems of general formula (II) comprising compounds of general formula (1).

## Description

### Field of the invention

The present invention relates to amine-substituted polycyclic compounds, which are fluorescence quencher dyes, to the processes of the preparation and to the use thereof as fluorescence quenchers, in particular in energy-transfer assays and applications. The present invention also relates to amine-substituted polycyclic compounds, which comprise bi-chromophoric systems.

### Background of the invention

For the detection of biological molecules or the investigation of biological processes and functions the use of fluorescent dyes or fluorescent proteins has evolved over the past two decades. As fluorescence can be detected with high sensitivity - even at single molecule level - this methodology gains a lot of attraction in research and industrial areas.

Without the use of fluorescent dyes, the development of high-throughput devices for DNA sequencing or flow cytometry would not have been possible. Beside the low detection limit another important advantage is the lower danger accompanied by minor measures concerning occupational health and safety compared to formerly used radioactive markers and detection methods.

With the increase of the usage of fluorescence for sensing and detection there was another principle moving back into the focus, i.e. the fluorescence resonance energy transfer or Förster resonance energy transfer named after his discoverer, the German physico-chemist Theodor Förster. This so-called FRET mechanism describes the radiationless energy transfer from one to another nearby molecule. The effectiveness of that process depends on the relative orientation of both molecules (i.e. orientation of their dipole moments), their distance and the spectral overlap, i.e. the energy of the involved molecular orbitals.

This energy transfer can happen between two different or identical molecules, i.e. intermolecular. But it is also possible to observe an intramolecular transfer between different parts of one molecule, e.g. within a bi-chromophoric system or in the case of so-called "tandem-dyes". These compounds are made up of two or more fluorophores and can exhibit a very large Stokes-shift, i.e. the wavelength-difference between absorption and emission.

This allows the simultaneous use of several dyes excited at the same wavelength but with increasingly larger Stokes-shifts expanding the capabilities of multiplexing applications.

With the FRET methodology the measurement of geometry (distance, size, arrangement of molecules or molecular groups, e.g. DNA hybridization and amplification) or reactivity (dynamics of protein folding, proteolytic degradation, enzyme activity, reaction speed, metabolic pathways etc.) and their changes over time are possible. An overview of bioanalytical applications of the FRET technology is given in K. Sapsford, L. Berti, I. Medintz, Materials for Fluorescence Resonance Energy Transfer Analysis, Angewandte Chemie International Edition 45, 2006, 4562-4588 (DOI: 10.1002/anie.200503873) or in A. Roda, M. Guardigli, E. Micheline, M. Mirasoli, Nanobiological luminenscence: Förster-type energy transfer methods, Anal. Bioanal. Chem. 393, 2009, 109-123 (DOI: 10.1007/s00216-008-2435-8).

What is the mechanism behind FRET and how can the observations be explained? At first the donor is electronically excited and then the energy is transferred from the donor to the acceptor, i.e. the fluorescence of the donor cannot be seen anymore. The acceptor can now either be a fluorescent or non-fluorescent molecule. In the first case the fluorescence of the acceptor could be observed, in the latter case there is no emission.

The acceptor in the second case is therefore called a fluorescence quencher, because it "deletes" or "erases" the fluorescence of the donor molecule. Such fluorescence quenchers for standard and novel applications in the ever-growing areas of life science and medicine are highly sought after, because beside their sole quenching ability these organic molecules need to have special features concerning their spectral quenching range, water solubility, aggregation behaviour, functional groups for chemical coupling, etc.

Important technical and industrial areas for the use of fluorescence quenchers are different PCR methods, which can e.g. be used in medical or forensic assays for the detection of cellular markers connected with cancer, metabolic disorders like diabetes and neurodegenerative diseases or other health threads like pandemic viruses, see S. Wadle, M. Lehnert, S. Rubenwolf, R. Zengerle, F. von Stetten, Real-time PCR probe optimization using design of experiments approach, Biomolecular Detection and Quantification 7, 2016, 1-8 (DOI: 10.1016/j.bdq.2015.12.002) or H. Zhu, H. Zhang, Y. Xu, S. Lassakova, M. Korabecna, P. Neuzil, PCR past, present and future, BioTechniques 69, 2020, 317-325 (DOI: 10.2144/btn-2020-0057).

Within the labelled oligonucleotides used in these applications the fluorescence of a donor is initially quenched by the non-fluorescent acceptor and can become visible after the cleavage or removal of the donor or acceptor from the oligonucleotide, i.e. through the increase of the distance between the donor and the acceptor the donor emission is eventually observed. A special design of such probes are the widely used "TaqMan"-probes used in quantitative PCR: The increase of the fluorescence signal during the thermal cycling is directly proportional to the fluorophore released and the amount of DNA template present in the PCR.

A similar approach is an oligonucleotide "hairpin probe" or "molecular beacon", in which a fluorophore and a quencher are in close proximity to each other due to a double stand formation of both ends. After adding a complementary target strand the loop is opened and the distance of both labels increases accompanied by the visibility of the initially quenched fluorescence of the donor.

Another important mechanism is photo-induced electron transfer (PET): The fluorescence of a fluorescent molecule is quenched via the transfer of an electron by an electron-rich molecule to the electronically excited fluorophore. This electron transfer can happen between two molecules, i.e. intermolecular, or intramolecular within the same molecule leading to a non-fluorescent compound.

Dye aggregation with the formation of H-type dimers leads also to the quenching of fluorescence. The direct physical contact of the two or more dye molecules in this case causes the dissipation of electronic energy into vibrational modes. This phenomenon is often observed in aqueous solution or solutions of high concentration of the dye molecules ("concentration quenching"). A similar effect may also be observed within a molecule consisting of more than one chromophore-units.

### Summary of the invention

It is therefore an object of the present invention to provide novel compounds for the use as fluorescence quenchers.

The object is achieved by compounds, dyes, uses, kits and methods as described hereinafter and in the preferred items and claims.

### Detailed description of the invention

The present invention relates to compounds having general formula (I)

Z-Y (I)
wherein Z is 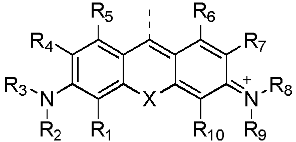
and Y is 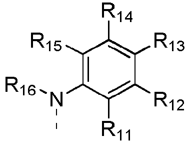
and Z and Y are joined together by a direct chemical bond indicated by the dashed lines,
wherein
R₁, R₄, R₅, R₆, R₇, R₁₀, R₁₁, R₁₂, R₁₃, and R₁₄ independently of one another are hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
R₂, R₃, R₈ and R₉ independently of one another are hydrogen or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O (aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
X is a group selected from CR₁₇R₁₈, SiR₁₉R₂₀, O, S, SO₂ and PO(OR₂₁), wherein R₁₇, R₁₈ , R₁₉ and R₂₀ independently of one another are hydrogen, CN, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃, or
R₁₇ and R₁₈ or R₁₉ and R₂₀, together with the atom to which they are attached, form a 3- to 7-membered ring, wherein the ring can comprise one or more double bonds; or/and one or more heteroatoms selected from the group consisting of N, O and S; or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
or/and wherein the 3- to 7-membered ring can be fused with one or more 3- to 7-membered rings, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms,
and wherein R₂₁ is hydrogen or a hydrocarbon group having from 1 to 20 carbon atoms;
or wherein R₁ and R₂, R₃ and R₄, R₇ and R₈ or/and R₉ and R₁₀, together with the atoms to which they are attached, form a 5- or 6-membered ring, wherein the ring can comprise one or more double bonds; and/or one or more heteroatoms selected from the group consisting of N, O and S; or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
and/or wherein at least one of R₁-R₁₄ forms a ring system with one or more neighbouring moieties,
and wherein R₁₅ and R₁₆ together with the atoms to which they are attached form a 4 - 8 membered ring, wherein this ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms or/and can be fused with one or more 3- to 6-membered rings, selected from cycloalkyl, aryl or heteroaryl, wherein these rings optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃,
and wherein R₂₂ and R₂₃ are independently defined as R₂.

The term "hydrocarbon group" as used within the context of the present application comprises a saturated or unsaturated hydrocarbon group having 1 to 40 carbon atoms, preferably 1 to 20 carbon atoms and more preferably 1 to 12 carbon atoms, which can be linear, branched or cyclic and has a bond valence at any one of the carbon atoms. Examples of hydrocarbon groups within the scope of the present invention include alkyl, cycloalkyl, alkenyl, cycloalkenyl, alkynyl, and aryl.

The hydrocarbon group may comprise one or more heteroatoms selected from the group consisting of N, O and S. Thus, a hydrocarbon group can also be a heteroalkyl, heterocycloalkenyl or heteroaryl group. A hydrocarbon group may comprise one or more substituents, preferably selected from a group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CON(alkyl)₂.

The term "alkyl" as used within the context of the present application refers to a saturated, linear or branched hydrocarbon group having from 1 to 40 carbon atoms, preferably having from 1 to 20 carbon atoms which has a bond valence at any one of the carbon atoms. Preferably, alkyl represents a hydrocarbon moiety having from 1 to 12 carbon atoms, more preferably having from 1 to 6 carbon atoms and most preferably having from 1 to 4 carbon atoms. Particularly preferred alkyls include methyl, ethyl, n-propyl, isopropyl, n-butyl, isobutyl, sec-butyl and tert-butyl.

The term "cycloalkyl" as used within the context of the present application refers to a saturated or unsaturated, cyclic hydrocarbon group having from 3 to 40 carbon atoms, preferably from 3 to 20 carbon atoms which has a bond valence at any one of the carbon atoms. Preferably, cycloalkyl represents a cyclic hydrocarbon group having from 3 to 12 carbon atoms, more preferably having from 3 to 8 carbon atoms. Particularly preferred cycloalkyls include cyclopropyl, cyclobutyl, cyclopentyl, cyclohexyl, cycloheptyl and cyclooctyl. In addition the cyclic hydrocarbon moieties can be interconnected and form bicyclic or polycyclic compounds, such as norbornane, norbornene, bicyclo[2.2.2]octyl, bicyclo[3.2.2]nonyl, and adamantyl.

The term "alkenyl" as used within the context of the present application refers to an unsaturated, linear or branched hydrocarbon group having from 2 to 40 caron atoms, preferably from 2 to 20 carbon atoms which has a bond valence at any one of the carbon atoms and at least one double bond. Preferably, alkenyl represents a hydrocarbon radical having from 2 to 12 carbon atoms, more preferably having from 2 to 6 carbon atoms. Particularly preferred alkenyls include ethenyl, propenyl and butenyl.

The term "alkynyl" as used within the context of the present application refers to an unsaturated, linear or branched hydrocarbon group having from 2 to 40 carbon atoms, preferably from 2 to 20 carbon atoms which has a bond valence at any one of the carbon atoms and at least one triple bond. Preferably, alkynyl represents a hydrocarbon group having from 2 to 12 carbon atoms, more preferably having from 2 to 6 carbon atoms. Particularly preferred alkynyls include ethynyl, propynyl and butynyl.

The term "aryl" as used within the context of the present application refers to an aromatic ring system having from 3 to 20 ring atoms, more preferably having from 6 to 14 ring atoms, which contains solely carbon atoms as ring atoms and has a bond valence at any carbon atom of the 3 to 20 ring-forming atoms. Preferred aryls include phenyl, naphthyl, anthracenyl and phenanthrenyl.

The term "heteroaryl" as used within the context of the present application refers to an aromatic ring system having from 3 to 20 ring atoms, more preferably having from 5 to 14 ring atoms, which contains as ring atoms, in addition to carbon atoms, at least one heteroatom selected from the group consisting of N, O and S and has a bond valence at any carbon atom or nitrogen atom of the 3 to 20 ring-forming atoms. Preferred heteroaryls include furanyl, imidazolyl, isothiazolyl, isoxazolyl, oxadiazolyl, oxazolyl, pyrazolyl, pyrrolyl, tetrazolyl, thiadiazolyl, thiazolyl, thienyl, triazolyl, pyrazinyl, pyridazinyl, pyridyl, pyrimidinyl and triazinyl. Alkyl, cycloalkyl, alkenyl and alkynyl groups as used within the context of the present application in a further preferred embodiment may comprise one or more heteroatoms selected from the group consisting of N, O and S and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CON(alkyl)₂.

The term "halogen" as used within the context of the present application includes fluorine, chlorine, bromine, and iodine, in particular fluorine and chlorine.

In the polycyclic compounds of the general formula (I) according to the invention, R₁ and R₂, R₃ and R₄, R₇ and R₈ or/and R₉ and R₁₀, together with the atoms to which they are attached, form in a preferred embodiment a 5- or 6-membered ring, 6-membered rings being more preferred. The at least one 5- or 6-membered ring can comprise one or more double bonds; and/or one or more heteroatoms selected from the group consisting of N, O and S; or/and one or more substituents, for example 1, 2, 3 or 4 substituents, which can themselves be identical or different.

The at least one 5- or 6-membered ring can in particular comprise one or more substituents, which substituents preferably are selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CON(alkyl)₂ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CON(alkyl)₂.

Particularly preferred as substituents of the at least one 5- or 6-membered ring are hydrocarbon groups having from 1 to 20 carbon atoms, in particular alkyl groups having from 1 to 6 carbon atoms, wherein the hydrocarbon groups in the case of a substitution preferably comprise one or more substituents selected from the group consisting of COOH, COO(alkyl), COO(aryl), SO₃H and CON(alkyl)₂.

Examples for such structures with additional rings include inter alia:

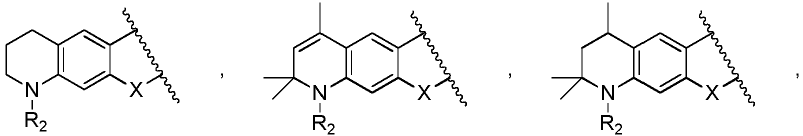

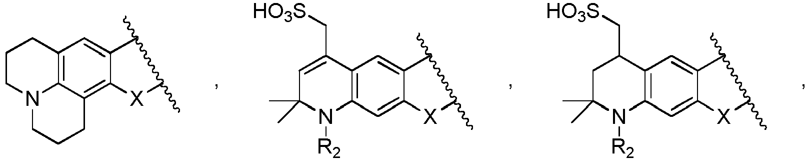

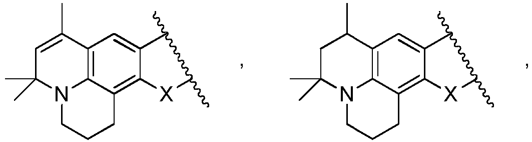

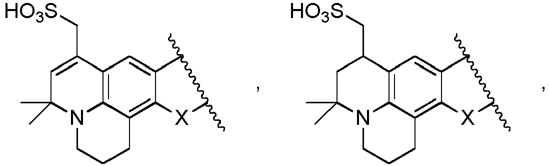

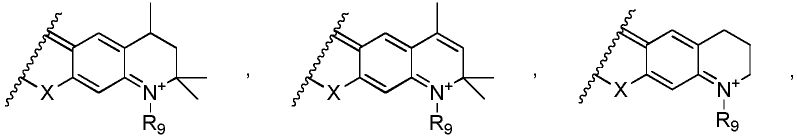

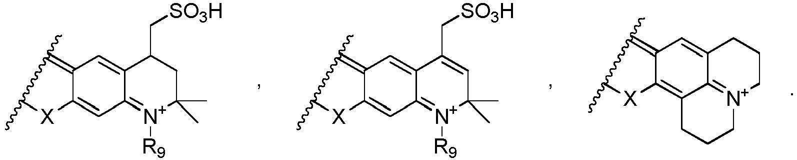

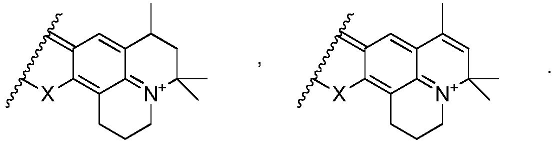

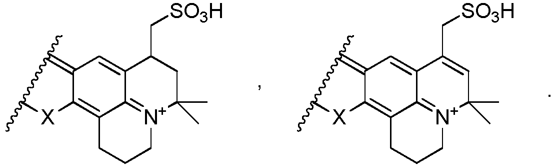

Further preferred embodiments are structures with annulated ring systems. They preferably result from (additional) ring-formation by R₄ and R₅ and/or R₆ and R₇. Preference is given to aromatic six-membered ring system as shown in the following examples:

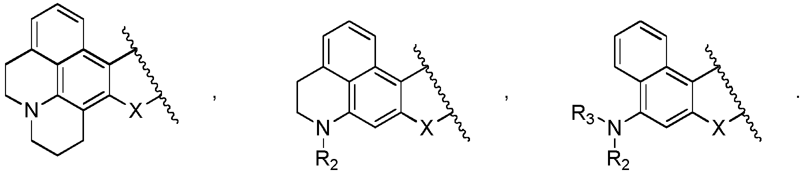

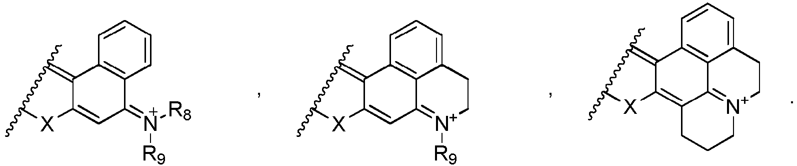

In a further preferred embodiment of the invention, the substituents R₂, R₃, R₈ or/and R₉ in the polycyclic compounds of the general formula (I) represent a hydrocarbon group having from 1 to 20 carbon atoms, wherein the hydrocarbon group optionally comprises one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CON(alkyl)₂. Particularly preferably, the hydrocarbon group is an alkyl group having from 1 to 6 carbon atoms, in particular methyl or ethyl. Preferred substituents are COOH and/or SO₃H.

COOH-groups are in particular suitable for coupling the inventive dyes, in particular for coupling the inventive dyes to a surface, to a carrier or to an analyte.

SO₃H-groups are in particular suitable to enhance water solubility of the compounds and/or to add negative charges to the compound, which can e.g. be advantageous in electrophoretic applications for the discrimination of differently charged molecules without changing their optical properties.

In a preferred embodiment the inventive compound of formula (I) comprises one or more COOH substituent(s) and/or one or more SO₃H substituent(s) at any position(s).

In a further preferred embodiment, the inventive compound of formula (I) comprises at least one functional group capable of covalent coupling, such as OH, SH, NH₂, COOH or a carboxylic acid derivative such as N-hydroxysuccinimidyl ester, alkene, such as trans-cyclooctene (TCO), alkyne such as dibenzocyclooctyne (DBCO), azide, tetrazine, tyramide or phosphoramidite. The coupling group is preferably comprised within group R₂, R₃, R₈ and/or R₉ and/or Y.

In a further preferred embodiment of the invention, at least two of R₁, R₅, R₆ and R₁₀, in particular R₅ and R₆, are hydrogen. Polycyclic compounds of the general formula (I), wherein R₁, R₅, R₆ and R₁₀ each representing hydrogen are particularly preferred.

In the polycyclic compounds of the general formula (I) according to the invention, X is a group selected from CR₁₇R₁₈, SiR₁₉R₂₀, O, S, SO₂ and PO(OR₂₁), with preference being given to CR₁₇R₁₈, SiR₁₉R₂₀, and O. More preferably, X is a group selected from CR₁₇R₁₈ and SiR₁₉R₂₀, most preferably CR₁₇R₁₈. If X is represented by the group CR₁₇R₁₈ or the group SiR₁₉R₂₀, at least one of R₁₇ and R₁₈ or R₁₉ and R₂₀ preferably represents a hydrocarbon group having from 1 to 20 carbon atoms, preferably an alkyl group having from 1 to 6 carbon atoms, more preferably methyl or ethyl.

Most preferred, X = C(CH₃)₂.

According to the invention R₁₅ and R₁₆ together with the atoms to which they are attached form a 4 - 8 membered ring, wherein this ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms or/and can be fused with one or more 3- to 6-membered rings, selected from cycloalkyl, aryl or heteroaryl, wherein these rings optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃.

According to certain embodiments of the invention, the compounds preferably comprise at least one SO₃H group. In addition, or alternatively, the compounds preferably comprise at least one carboxyl group. In addition or in the alternative, the compounds preferably comprise at least one group CONR₂₂R₂₃, in particular a group CON(CH₃)(CH₂-CH₂-CH₂-COOH).

Preferred embodiments are compounds, wherein Z in general formula (I) has one of the following structures (AA), (BB), (CC), (DD), (EE), (FF), (GG), (HH), (JJ), (KK), (LL), (MM), (NN), (OO), (PP), (QQ), (SS), (TT), (UU), (AAA), (BBB), (CCC), (DDD), (EEE), (FFF), (GGG), (HHH), (JJJ), (KKK), (LLL), (MMM), (NNN), (OOO), (PPP), (QQQ), (SSS), (TTT), (UUU), (AAAA), (BBBB), (CCCC), (DDDD), (EEEE), (FFFF), (GGGG), (HHHH), (JJJJ), (KKKK), (LLLL), (MMMM), or (NNNN):

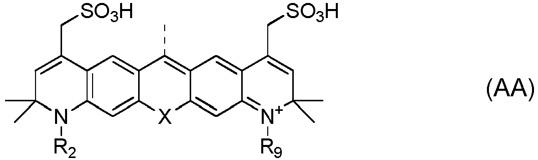

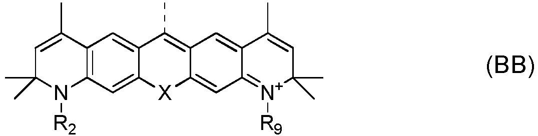

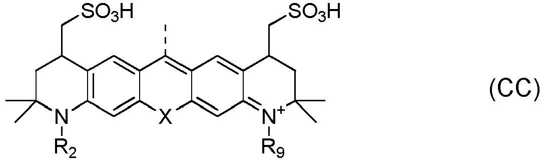

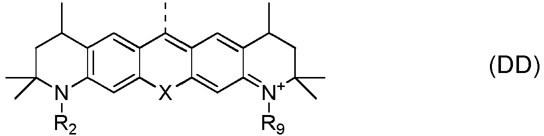

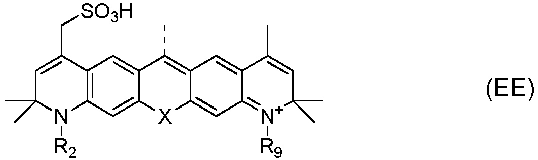

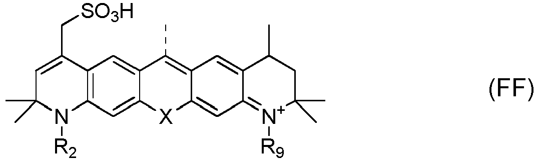

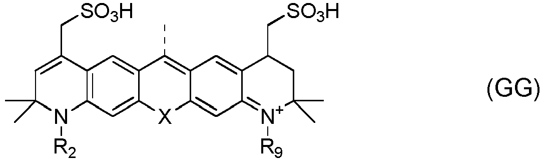

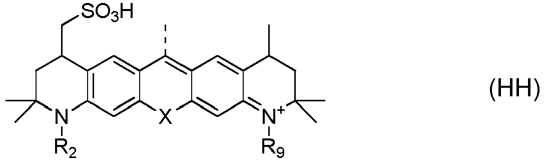

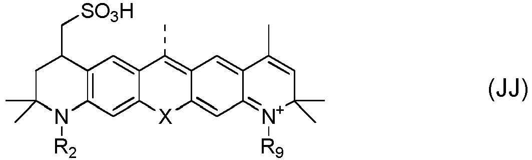

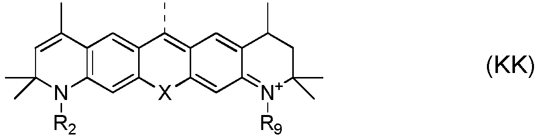

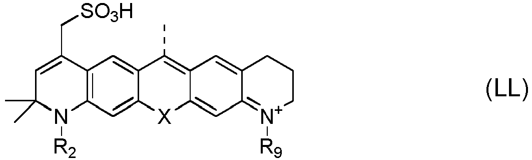

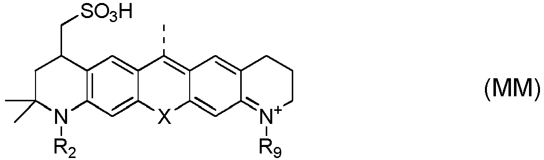

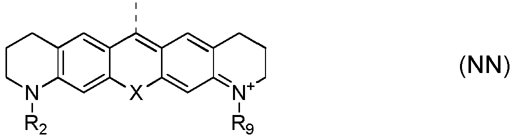

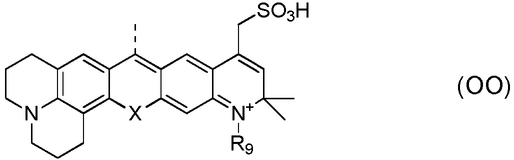

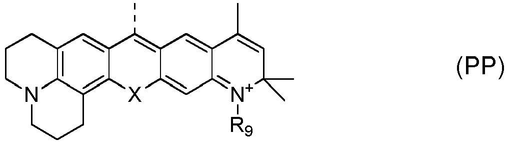

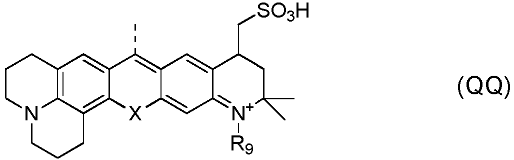

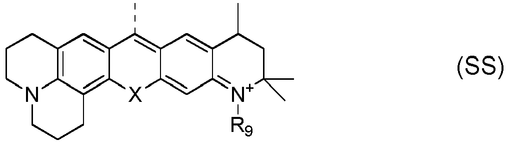

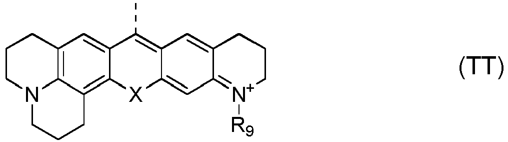

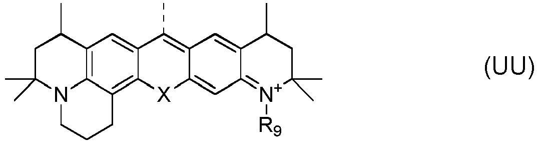

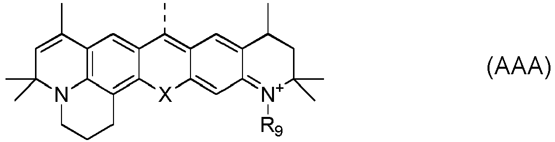

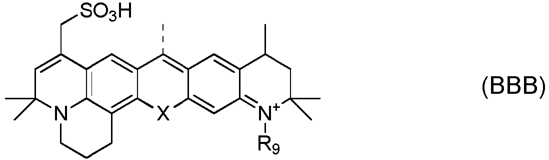

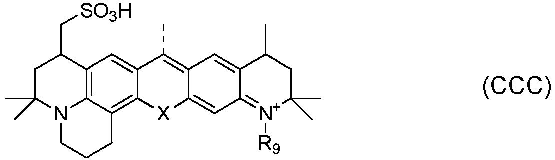

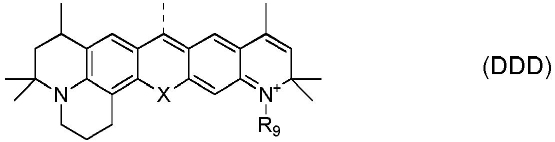

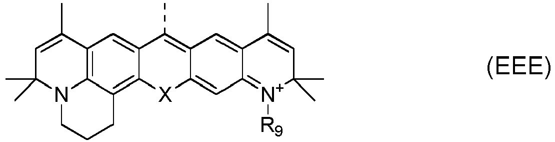

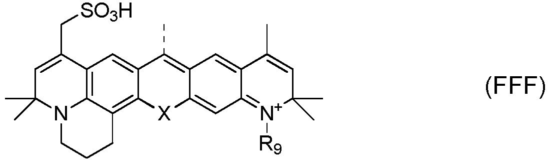

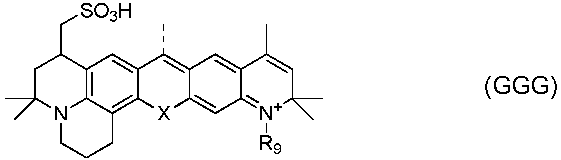

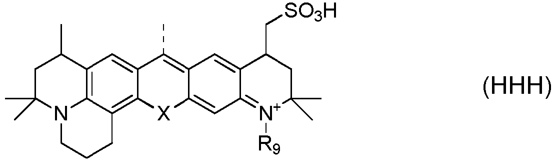

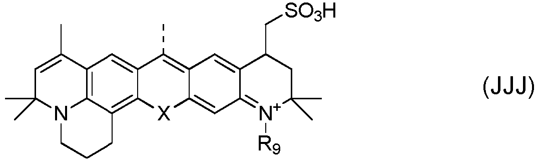

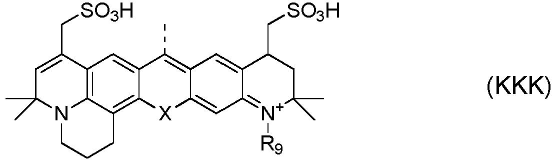

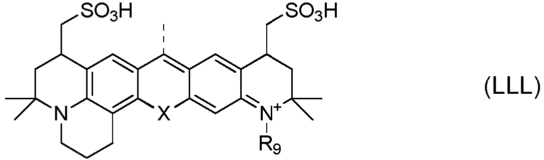

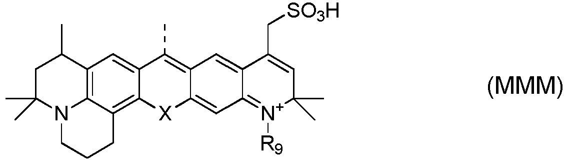

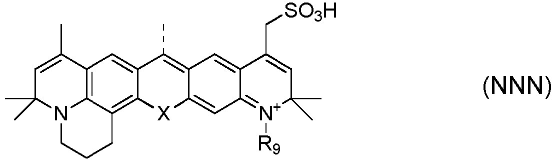

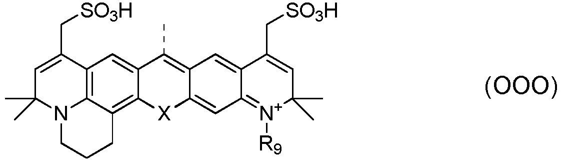

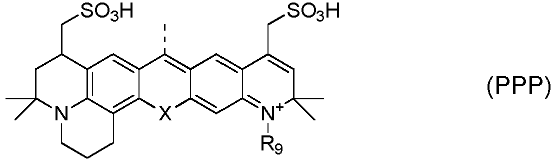

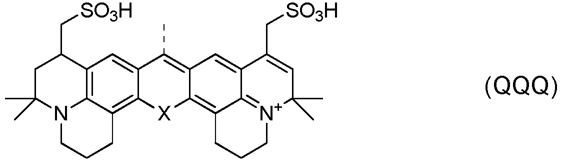

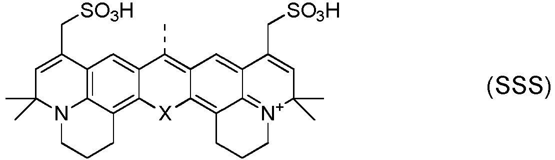

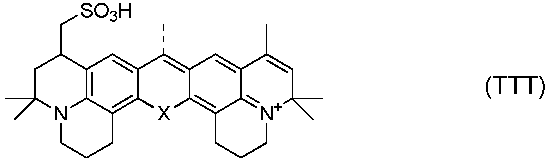

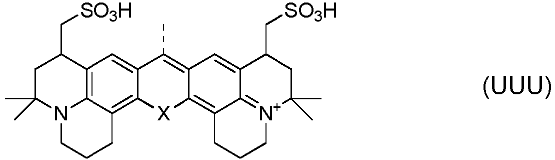

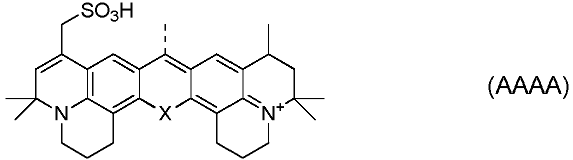

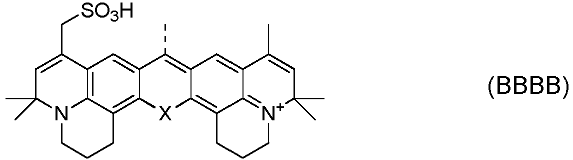

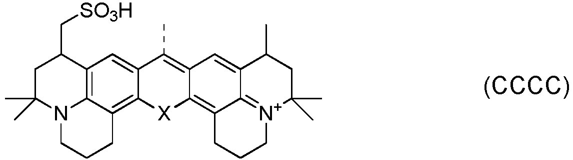

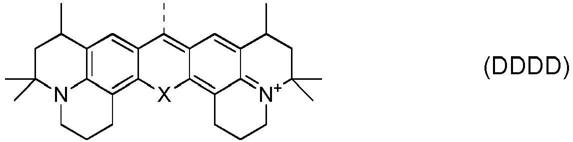

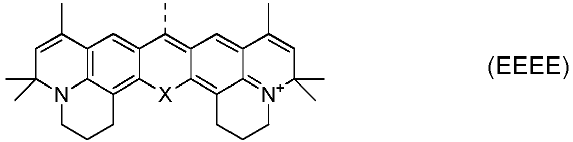

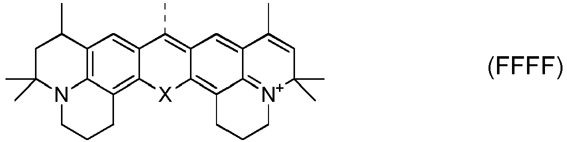

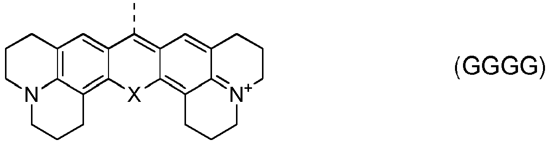

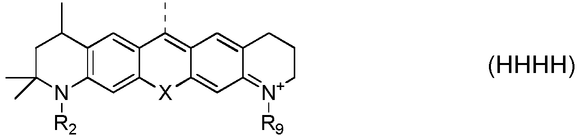

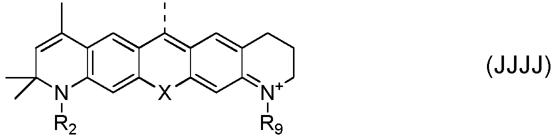

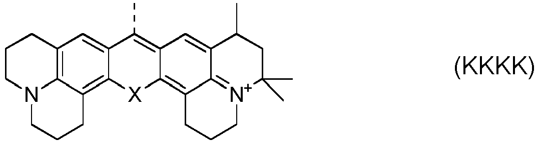

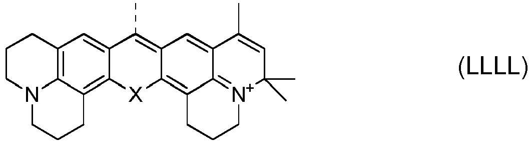

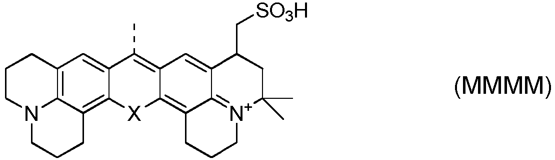

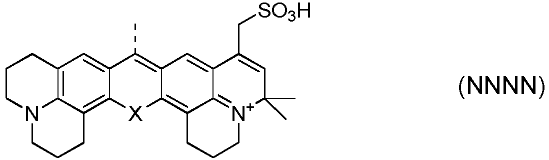

It is preferred according to certain embodiments of the invention that Y in general formula (I) has one of the following structures (A), (B), (C), (D), (E), (F), (G) or (H): 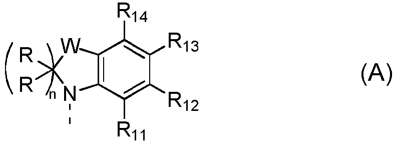 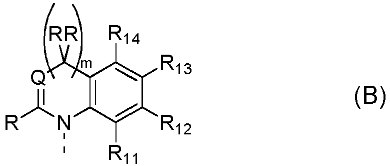 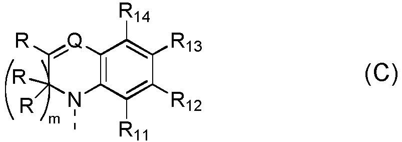 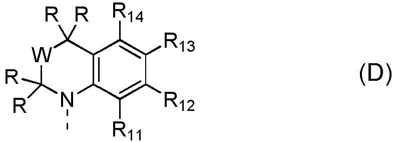 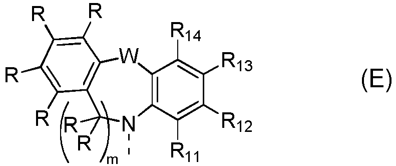 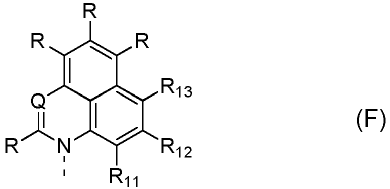 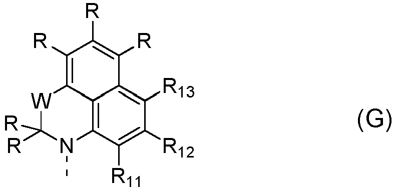 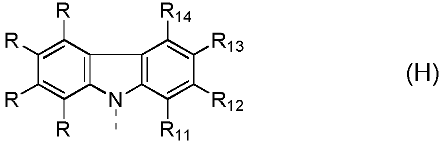 wherein
W = CR₂₄R₂₅, NR₂₆, ⁺NR₂₇R₂₈, S or O;
Q = CR₂₉, N or ⁺NR30;
n = 0, 1, 2, 3 or 4;
m = 0, 1, or 2;
R₂₄, R₂₅, R₂₇, R₂₈, R₂₉ and R₃₀ are independently defined as R₂;
R₂₆ is independently defined as R₂ or V;
V is a monovalent group;
and R at each occurrence is independently defined as R₁.

In a preferred embodiment, Y in general formula (I) has one of the structures (A), (D) (E) or G), wherein
W = NR₂₆;
n = 0, 1, 2, 3, or 4;
m = 0, 1, or 2;
R at each occurrence independently is defined as R₁ and
R₂₆ is independently defined as R₂ or V.

Preferably, V in formula (I) is a monovalent group as further defined herein with regard to formulae (II) and (III).

Preferred structures Y include:

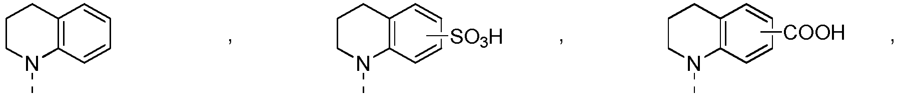

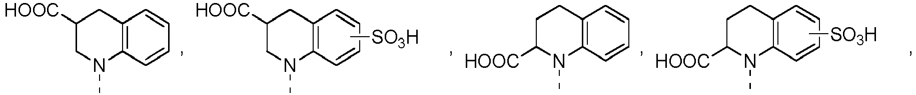

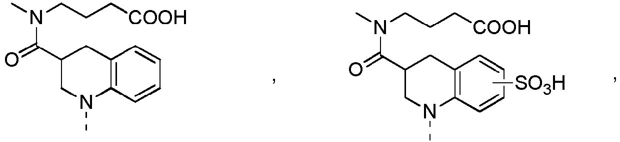

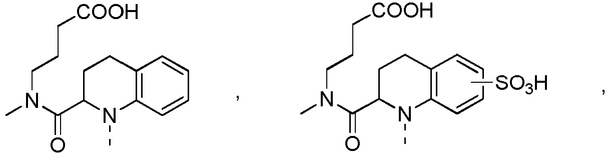

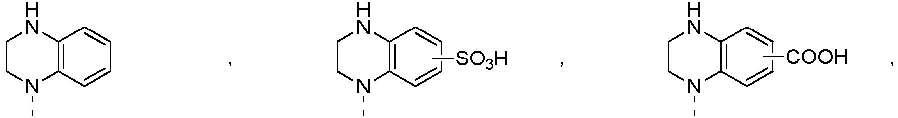

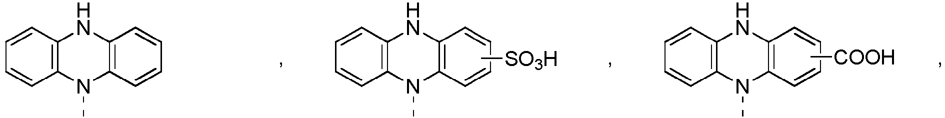

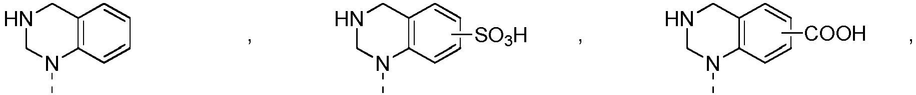

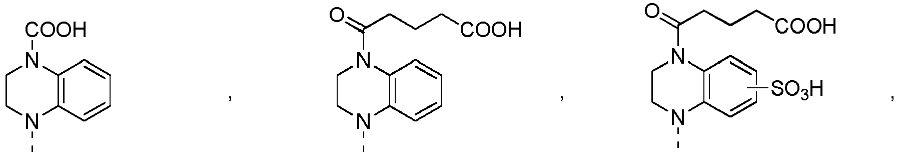

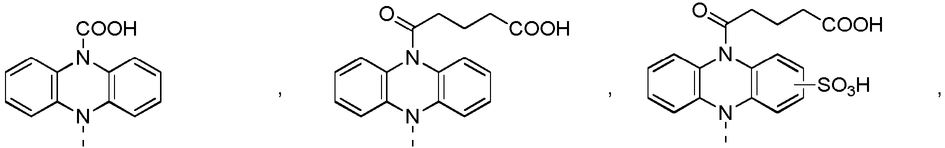

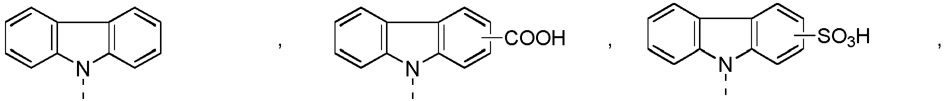

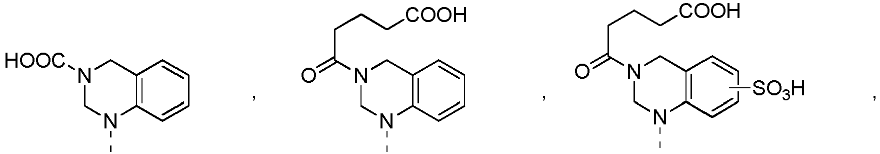

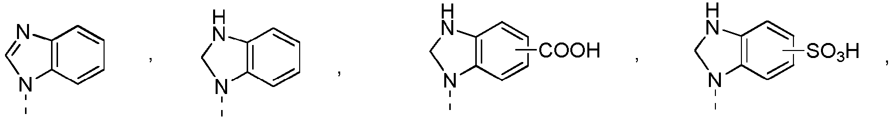

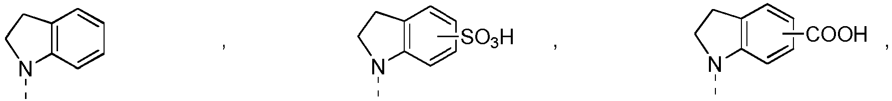

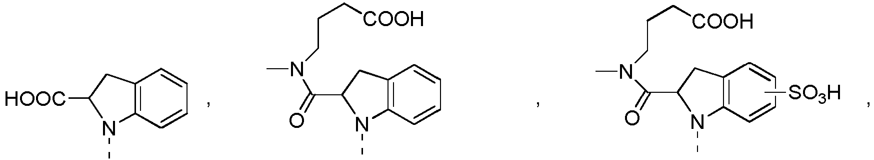

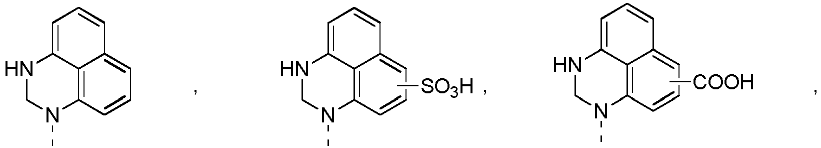

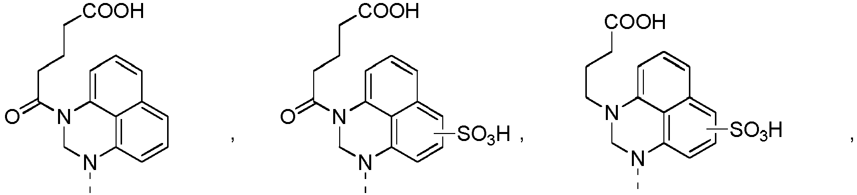

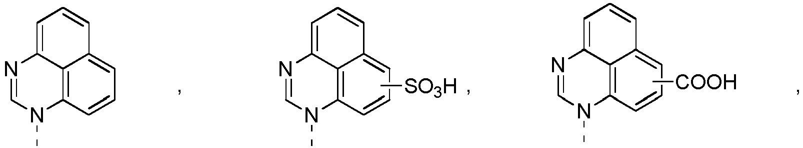

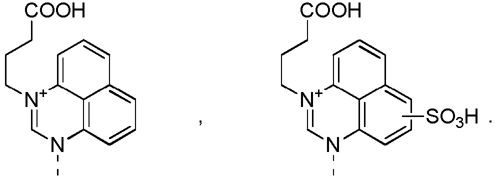

Inventive compounds of general formula (I), as well as or formulae (II) or (II) described below, are especially useful as fluorescence quenchers and/or for the qualitative or quantitative determination of an analyte in a sample or in energy-transfer assays and applications.

The inventive compounds in particular have an absorption maximum in the wavelength range from 500 nm to 800 nm, in particular from 600 nm to 750 nm. Thus, the compounds can be used as fluorescence quenchers for donors who fluoresce in the visible and near-infrared spectral region.

The inventive compounds have an absorption bandwidth - of which the "full width at half maximum" (FWHM) is a measure - of at least 40 nm FWHM. More preferable of 50 nm to 120 nm and even more preferable of 80 nm to 100 nm.

The inventive compounds have an extinction coefficient of at least 25.000 mol I⁻¹ cm⁻¹, more preferably of at least 50.000 mol I⁻¹ cm⁻¹ and even more preferably of at least 75.000 mol l⁻¹ cm⁻¹. The higher the extinction coefficient the better the absorption capability and therefore both the colorimetric detection and the quenching of the donor fluorescence.

The inventive compounds do not fluoresce, i.e. they have a fluorescence quantum yield below 1%, more preferably below 0,1% and even more preferably below 0,01%. This is the basic requirement for use as effective fluorescence quenchers.

The inventive compounds, further show a high photostability, comparable high as the photostability of the most photostable organic dyes and chromophores derived from the pyronin or carbopyronin system. Commercially available examples thereof on the market are e.g. Rhodamine 6G, ATTO 647N and ATTO 643. All of them are well known for their very high photostability.

The inventive compounds have excellent thermal stability, which makes them particularly suitable for use in all PCR devices and related applications.

The inventive compounds show a high pH-stability between pH 3 and pH 10, so that they are ideally suited for use in the physiological pH-range in life science applications.

In a preferred embodiment the present invention relates to a compound having general formula (II)

**Z-Y-V** (II),

wherein
Y has one of the structures (A), (D), (E) or (G); wherein
W = NR₂₆ and
R₂₆ = V, wherein V is a monovalent group.

V is a monovalent group and preferably a dye, an oligomer, a polymer, a sugar or polysaccharide residue, a peptide, a protein, a nucleotide, an oligonucleotide, a nanobody, an antibody, a hapten, a lipid or a macrocyclic host selected from cucurbiturils, calixarenes, cyclodextrins, cryptophanes, crown ethers or cryptands.

V preferably is a dye providing bi-chromophoric compounds. More preferably, V comprises a chromophoric structure or a fluorescent or non-fluorescent dye selected from coumarins, acridines, fluoresceins, anthraquinone-dyes, azo-dyes, nitro-dyes, quinonimine-dyes, indigoid dyes, di- or triphenylmethane dyes, dipyrrometheneboron difluoride dyes, cyanins, carbazines, triangulenium dyes, squarylium dyes, rosamines, rhodamines, pyronins, rhodols, carborhodamines, carbopyronins, carborhodols, silicorhodamines, silicopyronins, silicorhodols, oxazines, thiazines, safranins, and phthalocyanines.

In a further preferred embodiment, the inventive compound has general formula (II)

**Z-Y-V** (II)

wherein V has independently the meaning given for Z, wherein Z and V can have an identical structure or wherein Z and V can have different structures.

In such a preferred embodiment of the invention V is defined as Z leading to a bi-chromophoric system of general formula (III)

**Z₁-Y-Z₂** (III)

wherein
Z₁ and Z₂ are independently defined as Z, and
Z₂ can have the identical structure as Z₁ or Z₁ and Z₂ can have different structures.

The bi-chromophoric systems according to general formulae (II) or (III) can be used to enhance the important and useful properties of a single quenching moiety by further increasing the absorption and reducing the fluorescence quantum yield in the case where Z₁ and Z₂ comprise identical chromophoric systems.

In the case of different moieties Z₁ and Z₂ the quenching range, i.e. the spectral region where an effective quenching occurs, can be narrowed, extended or adjusted by the selection of appropriate moieties Z₁ and/or Z₂ comprising two different chromophoric systems.

From the compounds of general formulae (I), (II) and (III), ionic/charged salts can be obtained or counterions are needed for being electric neutral. These salts may possess an altered solubility in polar, particularly aqueous media. Apart therefrom, such salts are equivalent to the shown formulae.

Furthermore, the compounds can in solution be present in a solvated, particularly hydrated form, or can contain one or more molecules of crystal water. Crystalline or amorphous forms are also equivalent to the shown formulae.

Some compounds may have asymmetric carbon atoms as optical active centers or double bonds. The racemates, diastereomers, and geometric isomers resulting therefrom are all covered by the present invention.

The compounds can further have non-natural isotope ratios or even be radioactively labelled. These isotope variants, whether or not they are radioactive, are also covered by the shown formulae.

In yet a further aspect, certain embodiments of the invention relate to the use of a polycyclic compound of the general formula (I), or formula (II), or formula (III) according to the invention as a quencher and/or for the qualitative or quantitative determination of an analyte in a sample or in energy-transfer assays and applications.

The compounds have a high extinction coefficient and can therefore be used as colorimetric labels for a conjugated substance. They permit the detection by their visible-light absorption.

For these uses the inventive compounds preferably comprise a group which allows linkage of the compounds to an analyte or substrate, in particular a group which allows covalent linkage of the compound to an analyte or substrate. Therefore, preferred are inventive compounds having at least one functional group capable of covalent coupling, such as OH, SH, NH₂, COOH or a carboxylic acid derivative such as N-hydroxysuccinimidyl ester, alkene (e.g. trans-cyclooctene (TCO)), alkyne (e.g. dibenzocyclooctyne (DBCO)), azide, tetrazine or tyramide for covalently linking the compound to an analyte or substrate. Another preferred coupling group is the phosphoramidite modification, e.g. for use of the inventive compounds in cyclic or automated oligonucleotide synthesis. These groups, which allow linkage are preferably comprised by groups R₂, R₃, R₈ and/or R₉ and /or Y.

Therefore, a further embodiment of the present invention is a conjugate comprising a compound as described herein, coupled to a conjugated substance.

Typically, preferred conjugated substances are amino acids, peptides, proteins, tyramine, polysaccharides, nucleoside, nucleotides, oligonucleotides, nucleic acids, haptens, drugs, hormones, lipids, lipid assemblies, oligomers, polymers, macrocyclic hosts, cells, bacteria or viruses, antibodies or nanobodies.

In another embodiment the conjugated substance is an amino acid or a polymer of amino acids such as a peptide or protein. The amino acids can be natural or non-natural amino acids containing special side chains with artificial moieties useful for bio-orthogonal or "click" coupling reactions.

In yet another embodiment, the conjugated substance is a nucleic acid base, nucleoside, nucleotide or a nucleic acid polymer, including those that are modified with an additional linker or spacer for the attachment of the inventive compounds. Preferred nucleic acid polymer conjugates are labelled, single- or multi-stranded, natural or synthetic DNA or RNA, DNA or RNA oligonucleotides, or DNA/RNA hybrids.

In another embodiment, the conjugated oligonucleotides are aptamers for a specific target molecule, i.e. a metabolite, dye, hapten, or protein.

The quenching compounds of the invention are useful in any application where energy transfer from a fluorescent donor to a non-fluorescent acceptor takes place, provided that some spectral overlap exists between the emission of the doner and the absorption spectra of the quenching compound of this invention. It does not matter which photophysical mechanism causes the energy transfer in detail. Typically, a fluorophore is used in combination with the quenching compound in a method that detects a change in the quenching capability due to the changed distance between the two dyes.

This variation of the quenching, i.e. change in fluorescence of the donor, can be detected by all methods known in the art for standard fluorescence assays.

In another embodiment, the conjugated substrate is a biological polymer that is additionally labelled with at least one second fluorophore to form an energy-transfer pair.

The labelled conjugate can function as an enzyme substrate, and enzymatic hydrolysis of the substrate disrupts the energy transfer between the first and second dye accompanied by a partial or complete restoration of fluorescence. Alternatively, the conjugated substance itself is a fluorescent dye (such as green fluorescent protein and Phycobiliproteins). Such systems, among others, can therefore be used to detect and/or quantify structural disassembly in enzymatic systems like protease or nuclease activity, enzyme-mediated protein modification, immunoassays etc.

If the energy-transfer pair is present on the same or different substances and a change in the three-dimensional structure results in change in the distance between donor and acceptor either a fluorescence quenching or enhancement can happen. Such conformational changes can e.g. be observed during protein folding, drug delivery, and other cellular processes.

The energy-transfer pair can be conjugated to an oligonucleotide that displays efficient fluorescence quenching in its hairpin conformation, but there are several other techniques utilizing such energy-transfer pairs in similar oligonucleotide-based assays and applications, like endonuclease restriction primers, sequence specific primers etc.

The methods can be carried out in any medium. The compounds according to the invention can be modified by non-polar or polar substituents for a better solubility in organic or aqueous solvents, respectively. The typical solvents for biological applications are water, aqueous buffer solutions or aqueous-miscible solutions.

In another embodiment of the invention, the compound of general formula (I), (II) or (III) can be combined with an appropriate fluorophore in an "energy-transfer pair"-kit, which can also comprise additional reagents, buffers, purification media, substrates like antibodies, oligonucleotides, for carrying out an assay.

Table 1 shows examples of particularly preferred polycyclic compounds of general formula (I) wherein Z is having a carbopyronin structure. These examples serve to give a better understanding of the present invention but are not intended to limit the scope of the invention.

**Table 1: Compounds according to general formula (I) with X = CR₁₇R₁₈**

| Solvents | (a) = Ethanol + 0,1 Vol.-% trifluoracetic acid | | |
|---|---|---|---|
| | (b) = Phosphate buffered saline (PBS), pH 7.4 | | |
| **Compound** | **Structure** | **λ_{abs} [nm]** | **FWHM [nm]** |
| 1 | 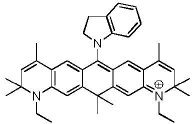 | 625 (a) | 102 (a) |
| 2 | 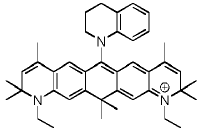 | 646 (a) | 91 (a) |
| 3 | 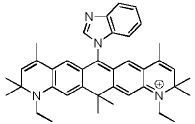 | 705 (a) | 42 (a) |
| 4 | 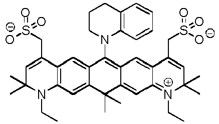 | 630 (b) | 87 (b) |
| 5 | 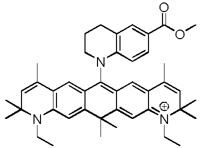 | 690 (a) | 55 (a) |
| 6 | 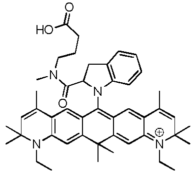 | 673 (a) | 103 (a) |
| 7 | 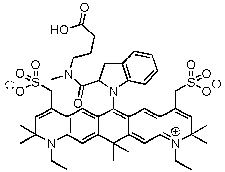 | 673 (b) | 103 (b) |
| 8 | 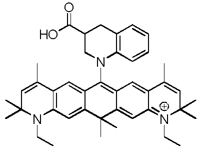 | 628 (a) | 103 (a) |
| 9 | 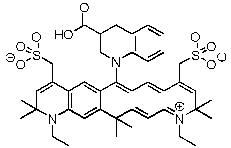 | 639 (b) | 91 (b) |
| 10 | 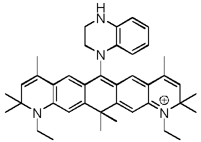 | 620 (a) | 86 (a) |
| 11 | 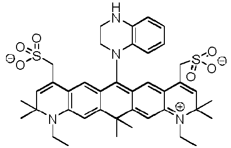 | 621 (b) | 86 (b) |
| 12 | 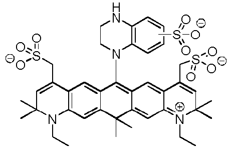 | 621 (b) | 86 (b) |
| 13 | 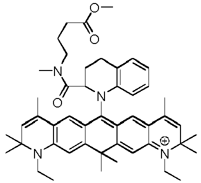 | 684 (b) | 82 (b) |
| 14 | 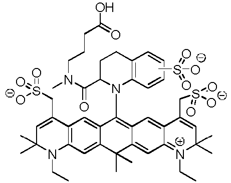 | 681 (b) | 69 (b) |
| 15 | 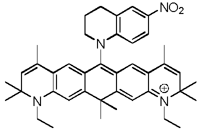 | 693 (b) | 43 (b) |
| 16 | 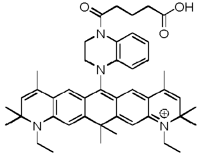 | 692 (a) | 48 (a) |
| 17 | 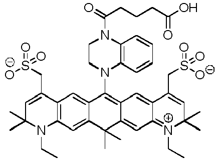 | 687 (b) | 52 (b) |
| 18 | 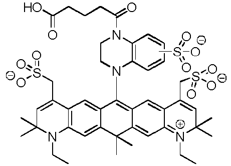 | 686 (b) | 52 (b) |
| 19 | 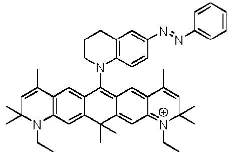 | 687 (a) | n.d. |
| 20 | 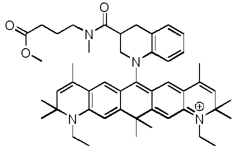 | 654 (a) | 85 (a) |
| 21 | 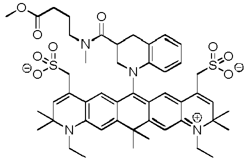 | 648 (b) | 84 (b) |
| 22 | 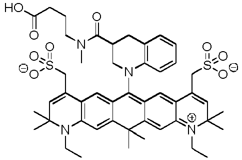 | 648 (b) | 84 (b) |
| 23 | 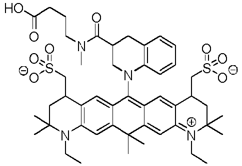 | 618 (b) | 82 (b) |
| 24 | 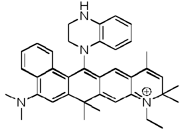 | 620 (a) | 105 (a) |
| 25 | 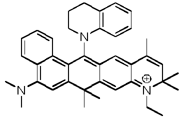 | 639 (a) | 111 (a) |
| 26 | 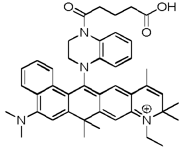 | 692 (a) | n.d. |
| 27 | 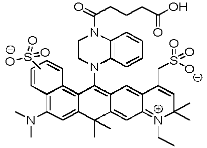 | 687 (b) | n.d. |

| | | | |
|---|---|---|---|
| n.d. = not determined | | | |

Usually, carbopyronin or carborhodamine dyes exhibit a high fluorescence quantum yield. Compounds of this chemical structure are described in US 6,828,159 B1 and several are sold by ATTO-TEC GmbH, Germany, as fluorescent labels, e.g. ATTO 610, ATTO 620, ATTO 633, ATTO 643, ATTO 647, and ATTO 647N. ATTO 643 and ATTO 647N are known as two dyes with the highest fluorescence quantum yield in the red region of the visible spectrum at around 650 nm, i.e. 62% and 65%, respectively. In comparison with the famous cyanine dyes Alexa Fluor 647 (33%) and Cy 5 (27%) the quantum yield is at least two times higher.

Also, carbopyronin dyes with an amine substituent at the central carbon atom - as described in US 9,651,490 B2 - show a very high quantum yield and additionally a very large stokes shift, making them ideal as fluorescent labels for multiplexing applications. A commercially available example is ATTO 490LS from ATTO-TEC GmbH, Germany, with a quantum yield of 30% in aqueous solution.

All the above given values for the fluorescence quantum yield of commercial fluorophores are taken from Appendix E of T. B. Ledur Kist, Fluorescent Dye Labels and Stains, John Wiley & Sons, 2023, DOI: 10.1002/9781119835165.

Therefore, it was completely unexpected and surprising that compounds of general formula (I) do not show any fluorescence or have a fluorescence quantum yield much smaller than 1%. For example, compound 4 has a quantum yield of 0,08% and compound 9 of about 0,04%; respectively. The quantum yield was determined in PBS buffer. This makes these compounds ideal candidates for the use as fluorescence quenchers in typical applications.

In contrast to compounds described in US 9,651,490 B2 without ring-closure of analogous positions to R₁₅ and R₁₆ in formulae (I), (II), or (III), the inventive compounds show firstly a pH-independent absorption and secondly a very good pH-stability.

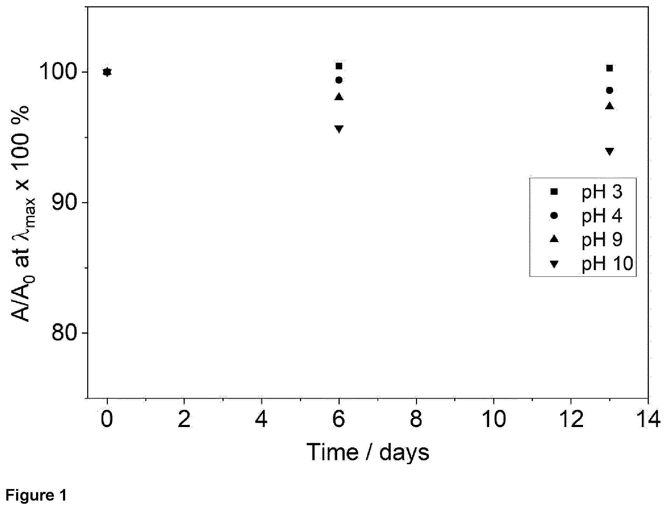

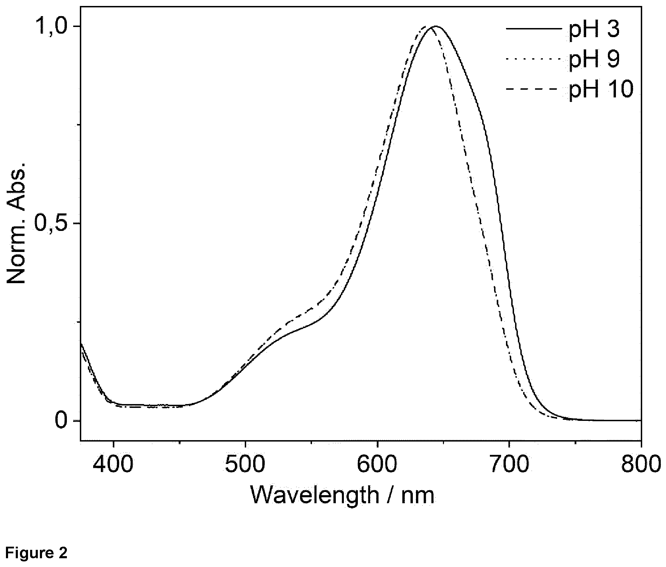

Figure 1 shows the excellent stability of compound 9 in aqueous buffer solutions of different pH ranging from pH 3 to pH 10 and Figure 2 shows the absorption spectra of compound 9 at different pH in acidic and alkaline solutions, respectively.

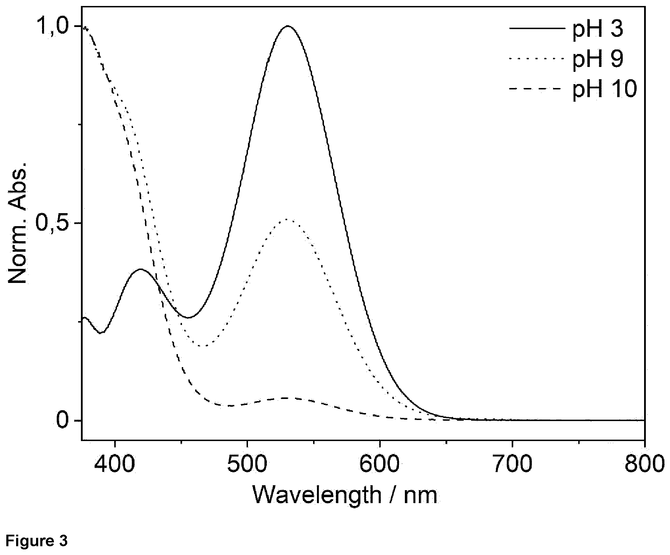

For comparison purposes we choose the following structure of the prior art 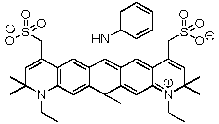 as a water-soluble comparison compound (without a cyclic structure involving the amine-N of moiety Y binding to Z) throughout the further discussion of the advantageous properties of the inventive compounds. With this comparison compound a strong change in absorption is visible in accordance with a change in pH (Figure 3).

The compounds of the prior art are very prone to degradation by the attack of hydroxyl ions in alkaline media. Hydroxyl ions are present at neutral pH and at alkaline pH, e.g. used during coupling reactions of the inventive compounds with biological molecules.

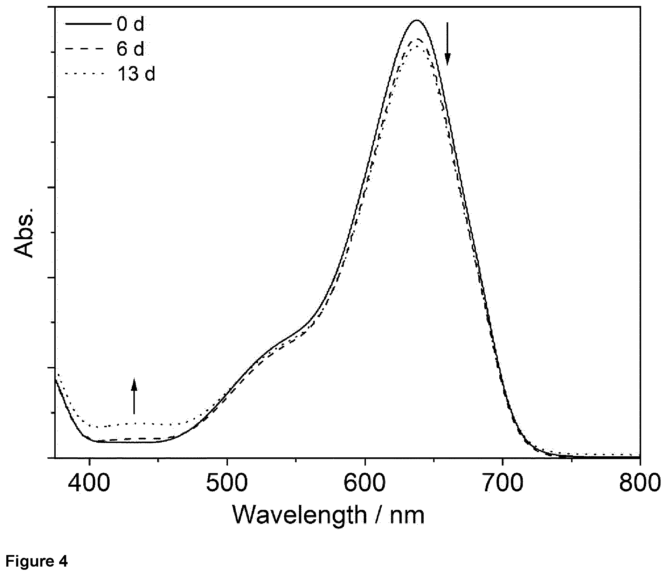

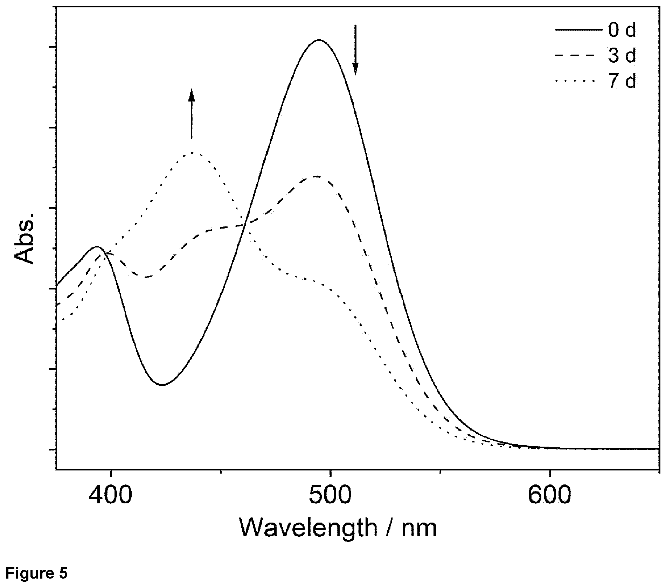

Furthermore, in alkaline media a highly fluorescent degradation product could be built as can be seen by a comparison of the absorption spectra of the not ring-closed compound 4 (AZ 285) from US 9,651,490 B2 with the ring-closed compound 9 of the present disclosure. While the absorption spectrum of the inventive compound 9 (Figure 4) remains almost unchanged for two weeks in Puffer pH 10 the absorption spectrum of the not ring-closed compound 4 (AZ 285) changes dramatically within a few days (Figure 5). The attack of hydroxyl ions followed by oxidation leads to compound 36 (AZ 35-A) from U S9,651,490 B2, which is a highly fluorescent anthrone derivative. Its strong fluorescence interferes with either the quencher properties or the original fluorescence signal of the parent compound.

An additional advantage of the inventive compounds is the possibility of having the coupling group at moiety Y of general formula (I), (II), or (III), e.g. as carboxylic acid group in compounds 6, 7, 8, 14 etc. (Table 1). If, after coupling the quencher compound according to the invention to a biomolecule, the unlikely event of a nucleophilic attack with the subsequent formation of a fluorescent reaction product occurs, this causes the chromophore (moiety Z) to be cleaved off. Afterwards that interfering fluorescent product can be removed in a simple washing step and no undesired fluorescence occurs during the further experiment.

The inventive compounds are very pH-stable, in particular over a pH range from 3 to 10, and therefore overcome these relevant und unpleasant disadvantages of the prior art.

Another important advantage of these compounds is their high extinction coefficient of about 75.000 I mol⁻¹ cm⁻¹ which exceeds that of usually used azo-based quencher dyes by almost two times, e.g. the extinction coefficient εₘₐₓ of compound 1 is 64.000 I mol⁻¹ cm⁻¹, of compound 2 is 75.000 I mol⁻¹ cm⁻¹ and of compound 10 is 66.000 I mol⁻¹ cm⁻¹, respectively.

The very well-known and widely used "Black Hole Quenchers" (BHQ) are azo-dyes with an extinction coefficient of 34.000 I mol⁻¹ cm⁻¹ (BHQ-1), 38.000 I mol⁻¹ cm⁻¹ (BHQ-2), and 42.700 I mol⁻¹ cm⁻¹ (BHQ-3). These values are taken from Table 3 in B. Fang, Y. Shen, B. Peng, H. Bai, L. Wang, J. Zhang, W. Hu, L. Fu, W. Zhang, L. Li, W. Huang, Small-Molecule Quenchers for Förster Resonance Energy Transfer, Angewandte Chemie International Edition 61, 2022, e202207188 (DOI: 10.1002/anie.202207188). From this table it becomes clear that even rhodamine-based quenchers, e.g. QSY7, QSY9, QSY21, due to their broader absorption bands have extinction coefficients of only about 90.000 I mol⁻¹ cm⁻¹. It can be seen from the FRET equation that a higher extinction coefficient would lead to a higher absorption and thus to a better acceptor ability of the excitation energy transferred from the donor.

The absorption spectra of typical quenchers are very broad due to their chemical nature of being e.g. azo dyes. Only a small influence of additional groups attached to the base chromophores can be observed, and, therefore, the absorption maximum is mainly given by that basic structure. This leads to only three to four quenchers covering the whole visible region of the electromagnetic spectrum.

In stark contrast to that, a very exact fine tuning of the absorption properties, i.e. maximum and bandwidth of the main absorption band, can be achieved by chemical modifications of compounds of general formula (I). This unique ability allows the use of the compounds according to the invention as quenchers for the selective quenching of a narrower wavelength range or distinct fluorophore, which is e.g. very helpful in the fast-growing area of multiplexing applications. This outstanding property is related to the chemical structure of the entire chromophore (moiety Z together with moiety Y), whose absorption can be influenced more sensitively by attaching side groups to moiety Z or altering the chemical nature of moiety Y.

The comparison of compounds 1, 2, and 3 shows a total bathochromic shift of the absorption maximum of about 80 only dependent on the chemical nature of the moiety Y. Another example is given by a comparison of compound 13 with compound 20, where the carboxamide-linker is attached at two different positions of the tetrahydroquinoline system leading to a difference in absorption of about 30 nm.

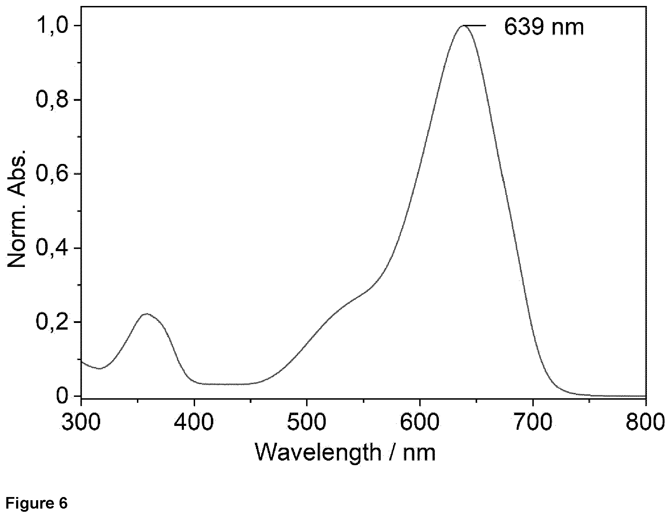

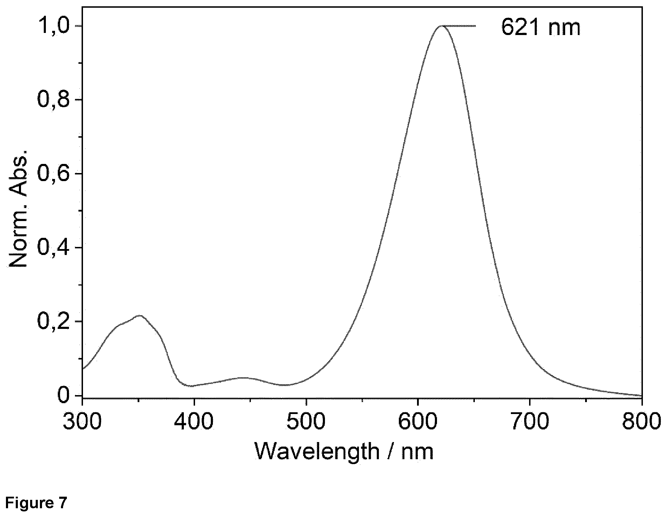

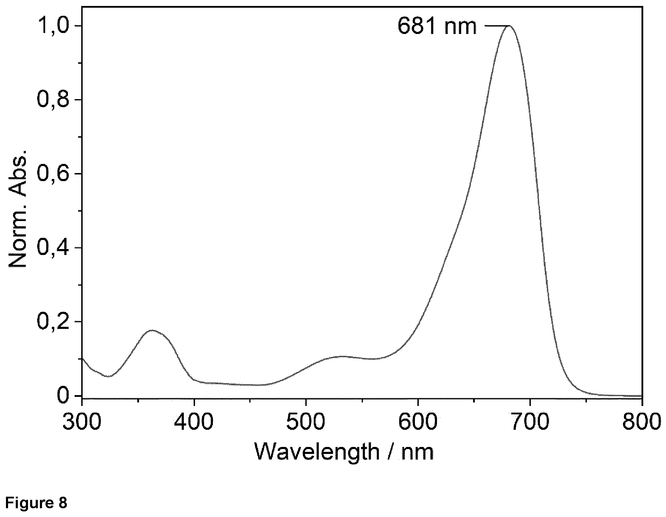

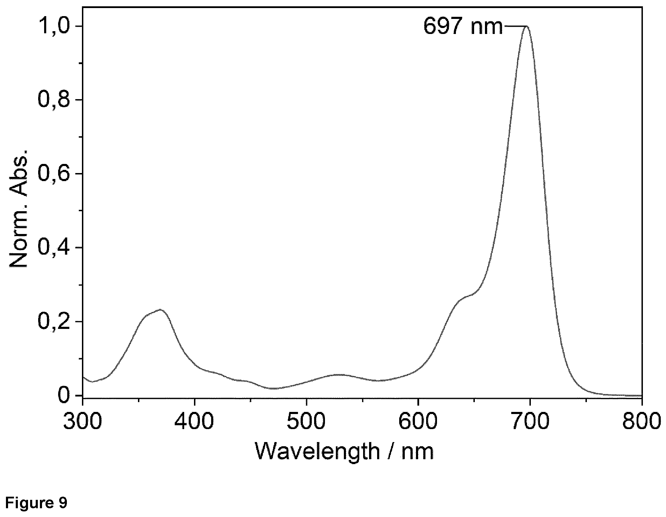

The absorption spectra of compounds 9, 12, 14, and 15 are given in Figures 6, 7, 8, and 9, respectively.

The hydrophilicity or solubility of the inventive compounds can be easily controlled by the introduction of hydrophilic or hydrophobic groups, like sulfonic acid groups, polyether chains or long-chain alkyl groups.

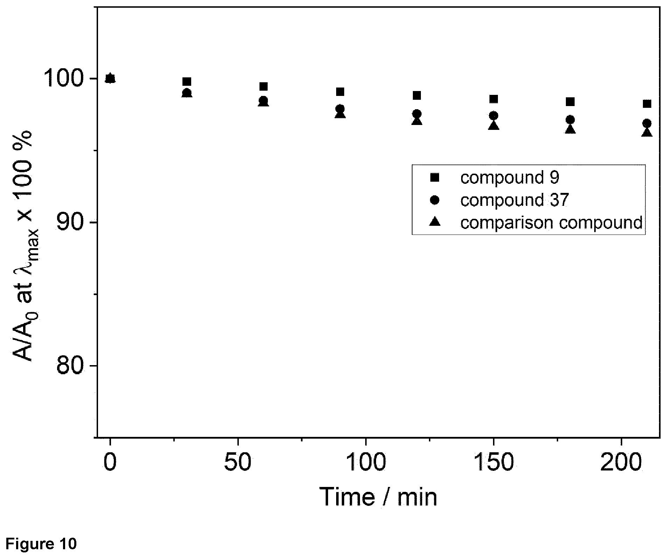

A further advantageous property is the high photostability of the inventive compounds. This is shown in Figure 10 for compounds 9, 37 and the comparison compound. The photostability - i.e. the time-dependent absorption changes at the long-wavelength absorption maximum of the compounds - was measured under stress conditions by irradiation of the compounds in aqueous buffer solutions with a 575 W mercury halide lamp.

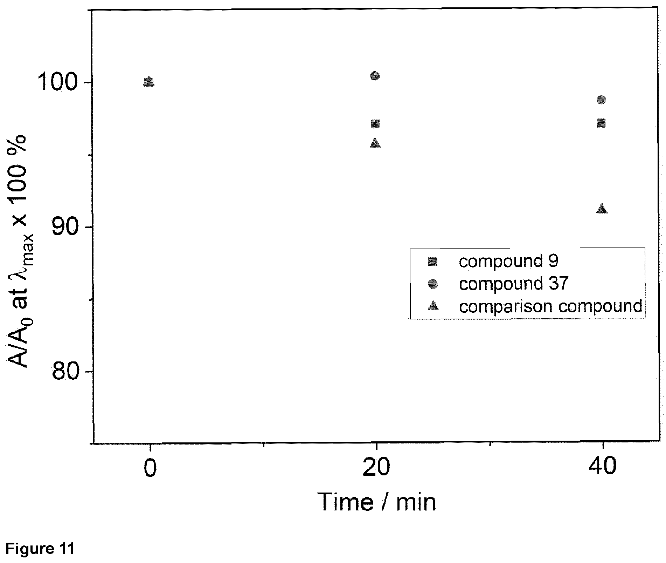

A widespread use of quencher dyes are PCR applications. During the reaction cycles the temperature is in- and decreased repeatedly. Therefore, it is important to use dyes of high thermal stability. It is common knowledge that an elevated temperature speeds up reactions and can therefore lead to a faster and more extensive destruction of the chromophoric systems. The inventive compounds where thus tested for their thermal stability (Figure 11): Solutions of the compounds 9, 37 and the comparison compound in commonly used PCR buffer (TRIS buffer mixed with magnesium chloride solution) were heated to 90°C for 40 minutes. No or negligible degradation could be observed by UV/VIS-spectroscopy for the inventive compounds, whereas the comparison compound already shows significant decomposition.

Tables 2, 3 and 4 show examples of inventive compounds of general formula (I) wherein Z is having a pyronin, thio-pyronin or silico-pyronin structure, respectively. These further examples serve to give a better understanding of the present invention but are not intended to limit the scope of the invention.

**Table 2: Compounds according to general formula (I) with X = O**

| Solvents | (a) = Ethanol + 0,1 Vol.-% trifluoracetic acid | | |
|---|---|---|---|
| | (b) = Phosphate buffered saline (PBS), pH 7.4 | | |
| **Compound** | **Structure** | **λ_{abs} [nm]** | **FWHM [nm]** |
| 28 | 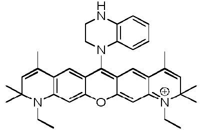 | 547 (a) | 69 (a) |
| 29 | 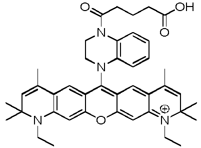 | 619 (a) | n.d. |
| 30 | 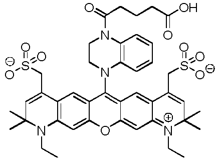 | 614 (b) | n.d. |

| | | | |
|---|---|---|---|
| n.d. = not determined | | | |

**Table 3: Compounds according to general formula (I) with X = S**

| Solvents | (a) = Ethanol + 0,1 Vol.-% trifluoracetic acid | | |
|---|---|---|---|
| **Compound** | **Structure** | **λ_{abs} [nm]** | **FWHM [nm]** |
| 31 | 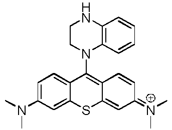 | 595 (a) | 78 (a) |
| 32 | 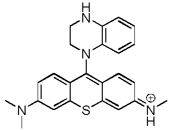 | 581 (a) | 75 (a) |

**Table 4: Compounds according to general formula (I) with X = SiR₁₉R₂₀**

| Solvents | (a) = Ethanol + 0,1 Vol.-% trifluoracetic acid | | |
|---|---|---|---|
| | (b) = Phosphate buffered saline (PBS), pH 7.4 | | |
| **Compound** | **Structure** | **λ_{abs} [nm]** | **FWHM [nm]** |
| 33 | 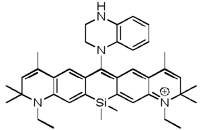 | 672 (a) | 109 (a) |
| 34 | 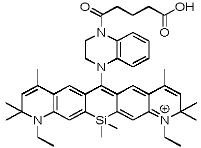 | 744 (a) | n.d. |
| 35 | 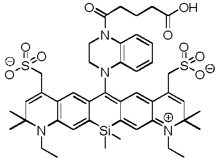 | 739 (b) | n.d. |

| | | | |
|---|---|---|---|
| n.d. = not determined | | | |

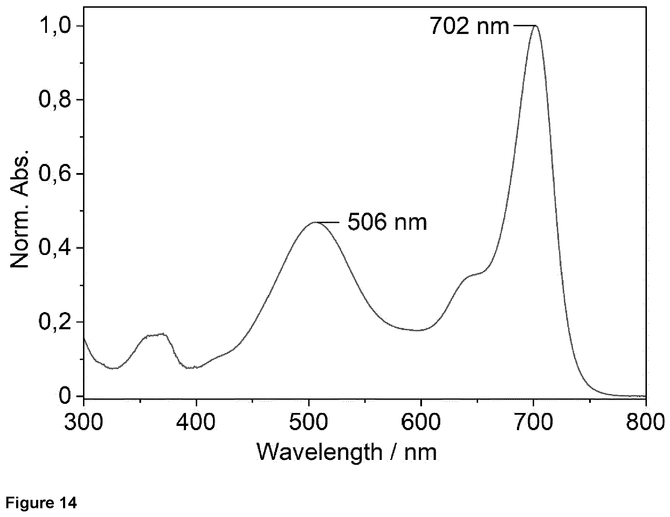

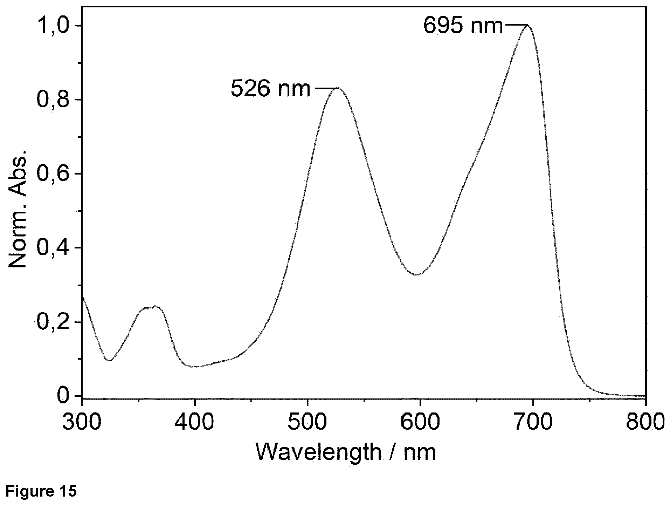

Table 5 shows examples of particularly preferred polycyclic compounds of general formulae (II) or (III). These examples of bi-chromophoric systems wherein Z₁ is having a carbopyronin structure serve to give a better understanding of the present invention but are not intended to limit the scope of the invention. If there are two different chromophoric systems present in compounds of general formula (II) or (III), two absorption bands with distinct absorption maxima are observed, see compounds 38 to 45 in table 5 and figures 14 and 15. Compounds 43 to 45 in table 5 have a carboxylic acid group, e.g. for coupling the inventive bi-chromophoric systems to a surface, to a carrier or to an analyte.

**Table 5: Compounds according to general formulae (II) or (III); bi-chromophoric systems**

| Solvents | (a) = Ethanol | |
|---|---|---|
| | (b) = Phosphate buffered saline (PBS), pH 7.4 | |
| **Compound** | **Structure** | **λ_{abs} [nm]** |
| 36 | 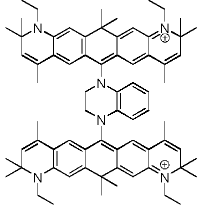 | 654 (a) |
| 37 | 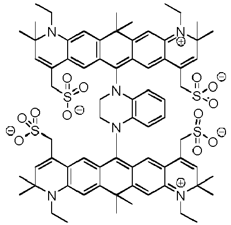 | 644 (b) |
| 38 | 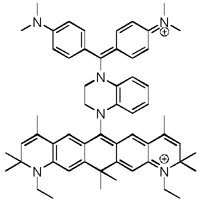 | 702 / 506 (a) |
| 39 | 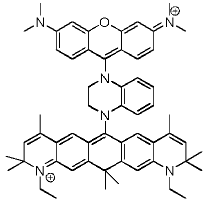 | 695 / 526 (a) |
| 40 | 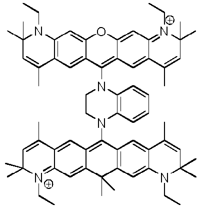 | 579 / 678 (a) |
| 41 | 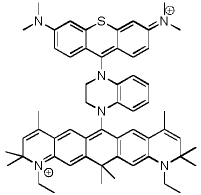 | 588 / 650 (a) |
| 42 | 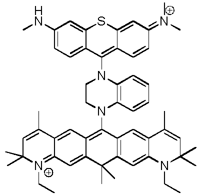 | 574 / 642 (a) |
| 43 | 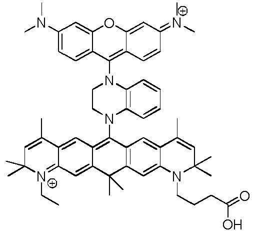 | 695 / 526 (a) |
| 44 | 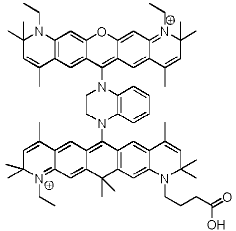 | 579 / 678 (a) |
| 45 | 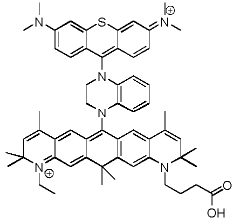 | 574 / 642 (a) |

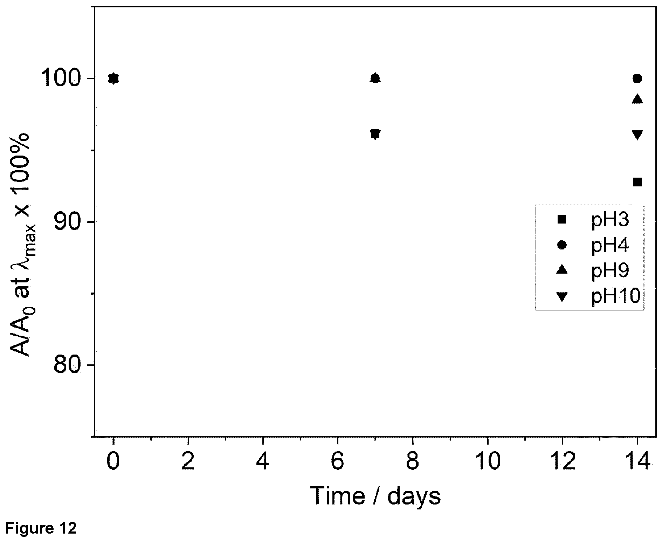

The bi-chromophoric compounds of the invention also have a very good pH-stability. Figure 12 shows the excellent stability of compound 37 in aqueous buffer solutions of different pH ranging from pH 3 to pH 10.

In these bi-chromophoric systems the important and useful properties of a single quenching moiety can be amplified by increasing the absorption and thus leading to a higher absorption rate of the energy transferred by a donor molecule. This becomes obvious by a comparison of the extinction coefficient εₘₐₓ of compounds 10 (εₘₐₓ = 66.000 l mol⁻¹ cm⁻¹) and of compound 36 (εₘₐₓ = 138.000 l mol⁻¹ cm⁻¹): In the bi-chromophoric system the extinction coefficient is more than doubled.

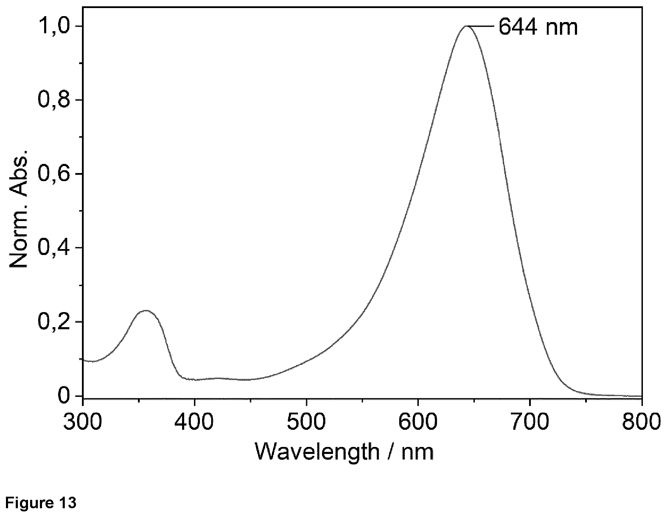

The absorption spectrum of compound 37 is shown in Figure 13.

In the case of different moieties Z₁ and Z₂ the quenching range, i.e. the spectral region where an effective quenching occurs, can be narrowed, extended or adjusted by the selection of appropriate moieties Z₁ and/or Z₂. This can be seen from the absorption spectra of compound 38 in Figure 14 and compound 39 in Figure 15, respectively: Each spectrum shows two absorption bands with different hight and width according to the properties of the two single chromophores combined into the inventive bi-chromophoric system.

An important mercantile advantage of the present invention is the simple and straightforward preparation of the compounds.

The precursors in the case of anthrones can be synthesised according to the procedures given in US 6,828,159 B1 and US 9,651,490 B2. These preparation methods can correspondingly be adopted for other chromophores, e.g. pyronins or silico-pyronins, by a person skilled in the art.

For the synthesis of several compounds of general formula (I) the same precursor of moiety Z can be used: This precursor is at first activated with trifluoromethanesulfonic acid anhydride (Tf₂O) and is than reacted in a simple procedure with different aromatic amines (moiety Y).

This is shown in the following reaction scheme for the synthesis of compound 8, for which 1,2,3,4-tetrahydroquinoline-3-carboxylic acid is used as moiety Y:

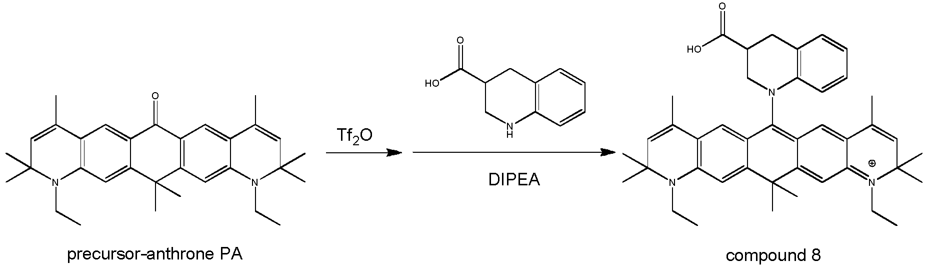

In the same way e.g. compounds 1, 2, 3, and 5 are easily prepared using the commercially available compounds indoline, 1,2,3,4-tetrahydroquinoline, 1H-benzimidazole, and methyl 1,2,3,4-tetrahydroquinoline-6-carboxylate, respectively.

If moiety Y has a second amine functionality the inventive bi-chromophoric systems can easily be prepared in a two-step synthesis as is described hereafter for compounds 36 and 38.

### Examples

### Examples for the preparation of compounds according to the invention

The precursor-anthrone PA (1,11-diethyl-2,2,4,8,10,10,13,13-octamethyl-1,2,10,11-tetrahydrobenzo[1,2-g:5,4-g']diquinolin-6(13H)-one) used in the following examples is prepared according to the procedures given in US 6,828,159 B1 and US 9,651,490 B2.

### Compound 8

A solution of PA (126 mg, 267 µmol) in dichloromethane (20 ml) is successively treated with trifluoromethanesulfonic acid anhydride (Tf₂O) (46,9 µl, 280 µmol) and with a solution of 1,2,3,4-tetrahydroquinoline-3-carboxylic acid (144 mg, 813 µmol) and N,N-diisopropylethylamine (DIPEA) (141 µl, 801 µmol) in dichloromethane (4 ml). The reaction mixture is stirred for 15 min at room temperature. Subsequently, the crude product is purified by column chromatography yielding compound 8 as blue solid (89,8 mg, 53%).

### Compound 10

A solution of PA (257 mg, 548 µmol) in dichloromethane (40 ml) is successively treated with Tf₂O (96 µl, 581 µmol) und 1,2,3,4-tetrahydroquinoxaline (195 mg, 1,45 mmol). The reaction mixture is stirred for 5 min at room temperature. Subsequently, the crude product is purified by column chromatography yielding compound 10 as blue solid (382 mg, 95%).

### Compound 20

A solution of compound 8 (211 mg, 337 µmol), 1-hydroxybenzotriazol (68,2 mg, 503 µmol), N,N'-dicyclohexylcarbodiimide (DCC) (126 mg, 605 µmol), methyl 4-(methylamino)-propanoate hydrochloride (225 mg, 1,34 mmol) und DIPEA (350 µl, 2,04 mmol) in dichloromethane (6 ml) is stirred for 16 h at room temperature. Subsequently, the crude product is purified by column chromatography yielding compound 20 as blue solid (143 mg, 160 µmol, 48%).

### Compound 22

A solution of compound 20 (137 mg, 154 µmol) in dichloromethane (4 ml) is treated with Oleum (7%, 800 µl). The solution is shaken until the organic phase becomes colorless and the reaction mixture is allowed to stand at room temperature for 72 h. Afterwards, the oleum phase is slowly poured on ice (20 g). To complete the hydrolysis of the methyl ester the resulting solution is adjusted to pH 10 with sodium hydroxide solution (5%) and stirred at 60°C for 20 h. The solution of the crude product is purified by column chromatography yielding compound 22 as blue solid (120 mg, 85%).

### Compound 36

A solution of PA (35,1 mg, 74,8 µmol) in dichloromethane (1 ml) is successively treated with Tf₂O (12,4 µl, 74,8 µmol), compound 10 (52 mg, 70,8 µmol) and DIPEA (12,4 µl, 71,1 µmol). Subsequently, the crude product is purified by column chromatography yielding compound 36 as blue solid (44 mg, 48%).

### Compound 37

A solution of compound 36 (16,8 mg, 12,6 µmol) in dichloromethane (ca. 0,5 ml) is treated with Oleum (5%, 400 µl). The solution is shaken until the organic phase becomes colorless and the reaction mixture is allowed to stand at room temperature for 40 h. Afterwards, the oleum phase is slowly added to ice water (33 ml). Acetonitrile (7 ml) is added to the mixture and the resulting solution is neutralized with sodium hydroxide solution (10%, 2 ml) and saturated sodium bicarbonate solution (4 ml). The crude product solution is purified by column chromatography yielding compound 37 as blue solid (3,0 mg, 17%).

### Compound 38

A solution of bis(4-(dimethylamino)phenyl)methanone (22 mg, 81,9 µmol) in dichloromethane (2 ml) is treated with Tf₂O (13,6 µl, 82 µmol). Compound 10 (57 mg, 78 µmol) and DIPEA (13,7 µl, 80 µmol) is added to the blue solution. Subsequently, the crude product is purified by column chromatography yielding compound 38 as violet solid (49 mg, 60%).

### Brief description of the drawings

**Figure 1****:** Stability of compound 9 in aqueous buffer solutions of different pH at 25°C.
**Figure 2****:** Absorption spectra of compound 9 at different pH at 25°C.
**Figure 3****:** Absorption spectra of comparison compound at different pH at 25°C.
**Figure 4****:** Change of the absorption spectrum of compound 9 for 13 days (buffer pH 10, 25°C).
**Figure 5****:** Change of the absorption spectrum of compound 4 (AZ 285) from US 9,651,490 B2 for 7 days (buffer pH 10, 25°C).
**Figure 6****:** Absorption spectrum of compound 9 in phosphate buffered saline (pH 7.4) at 25°C.
**Figure 7****:** Absorption spectrum of compound 12 in phosphate buffered saline (pH 7.4) at 25°C.
**Figure 8****:** Absorption spectrum of compound 14 in phosphate buffered saline (pH 7.4) at 25°C.
**Figure 9****:** Absorption spectrum of compound 15 in ethanol at 25°C.
**Figure 10****:** Photostability of compound 9, compound 37 and the comparison compound in phosphate buffered saline (pH 7.4) when irradiated with a 575 W mercury halide lamp.
**Figure 11****:** Thermal stability of compound 9, compound 37 and the comparison compound in PCR buffer at 90°C.
**Figure 12****:** Stability of compound 37 in aqueous buffer solutions of different pH at 25°C.
**Figure 13****:** Absorption spectrum of compound 37 in phosphate buffered saline (pH 7.4) at 25°C.
**Figure 14****:** Absorption spectrum of compound 38 in ethanol at 25°C.
**Figure 15****:** Absorption spectrum of compound 39 in ethanol at 25°C.

The present application also provides the following preferred items:
Item 1: A compound having general formula (I)

   Z-Y (I)

   wherein Z is 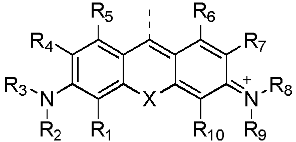 and Y is 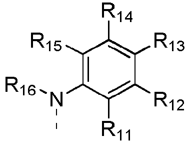 and Z and Y are joined together by a direct chemical bond indicated by the dashed lines,
   wherein
   R₁, R₄, R₅, R₅, R₇, R₁₀, R₁₁, R₁₂, R₁₃, and R₁₄ independently of one another are hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO3H, SO₂NH₂, SO₂NH(alkyl), SO₂NH(aryl), SO₂N(alkyl)₂, SO₂N(aryl)₂, and CONR₂₂R₂₃;
   R₂, R₃, R₈ and R₉ independently of one another are hydrogen or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O (aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃;
   X is a group selected from CR₁₇R₁₈, SiR₁₉R₂₀, O, S, SO₂ and PO(OR₂₁), wherein R₁₇, R₁₈, R₁₉ and R₂₀ independently of one another are hydrogen, CN, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃; or
   R₁₇ and R₁₈ or R₁₉ and R₂₀, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃;
   or/and wherein the 3- to 7-membered ring can be fused with one or more 3- to 7-membered rings, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms;
   and wherein R₂₁ is hydrogen or a hydrocarbon group having from 1 to 20 carbon atoms;
   or wherein R₁ and R₂, R₃ and R₄, R₇ and R₈ or/and R₉ and R₁₀, together with the atoms to which they are attached, form a 5- or 6-membered ring, wherein the ring can comprise one or more double bonds; and/or one or more heteroatoms selected from the group consisting of N,O and S; or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃,
   and/or wherein at least one of R₁-R₁₄ forms a ring system with one or more neighbouring moieties,
   and wherein R₁₅ and R₁₆ together with the atoms to which they are attached form a 4 - 8 membered ring, wherein this ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms or/and can be fused with one or more 3- to 6-membered rings, selected from cycloalkyl, aryl or heteroaryl, wherein these rings optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃,
   and wherein R₂₂ and R₂₃ are independently defined as R₂.
Item 2: The compound of item 1, wherein Y in general formula (I) has one of the following structures (A), (B), (C), (D), (E), (F), (G) or (H): 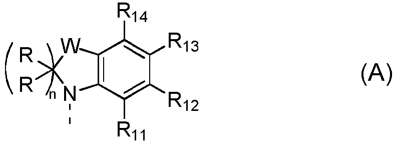 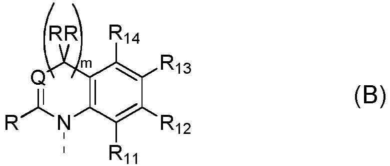 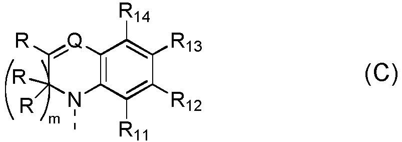 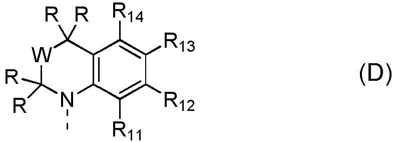 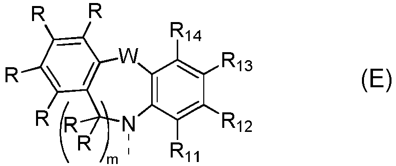 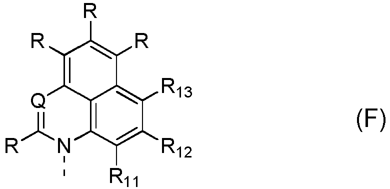 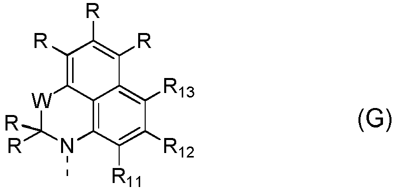 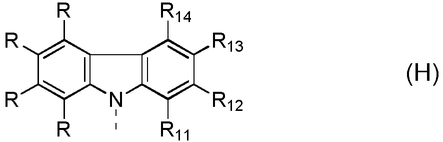 wherein
   W = CR₂₄R₂₅, NR₂₆, ⁺NR₂₇R₂₈, S or O;
   Q = CR₂₉, N or ⁺NR₃₀;
   n = 0, 1, 2, 3 or 4;
   m = 0, 1, or 2;
   R₂₄, R₂₅, R₂₇, R₂₈, R₂₉ and R₃₀ are independently defined as R₂;
   R₂₆ is independently defined as R₂ or V;
   V is a monovalent group;
   and R at each occurrence is independently defined as R₁.
Item 3: The compound of any one of items 1 or 2, wherein R₁ and R₂ and/or R₃ and R₄ form a ring system having one of the following structures: 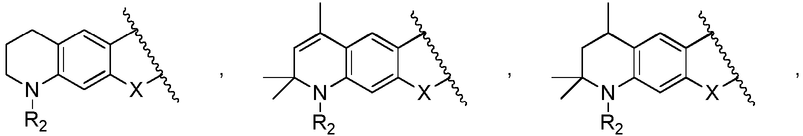 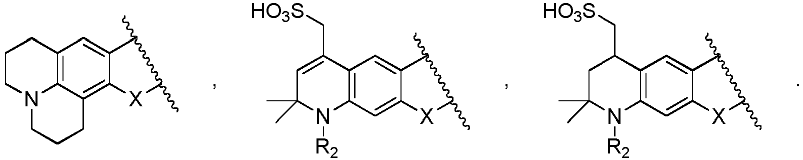 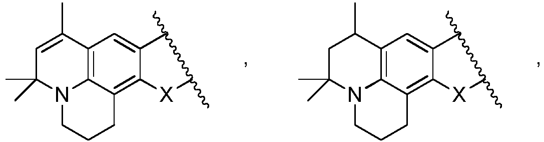 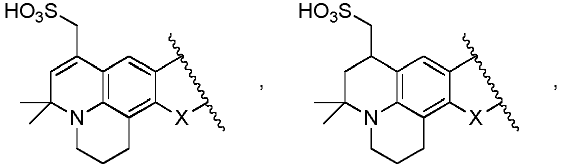 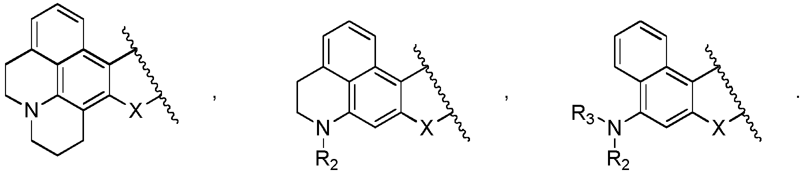
Item 4: The compound of any one of items 1 to 3, wherein R₇ and R₈ and/or R₉ and R₁₀ form a ring system having one of the following structures: 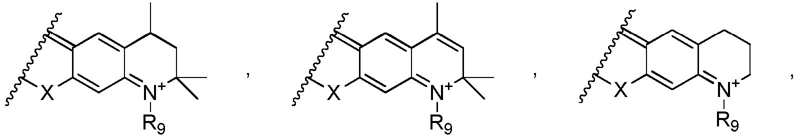 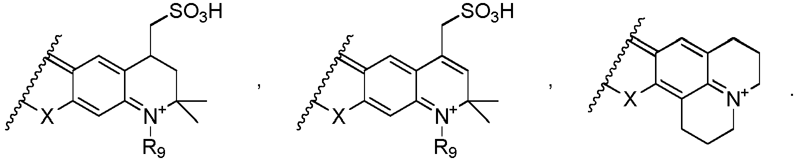 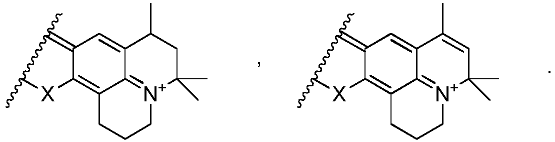 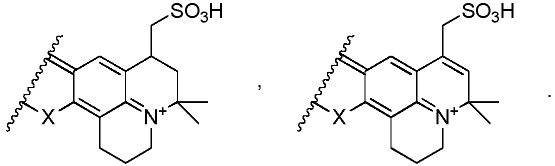 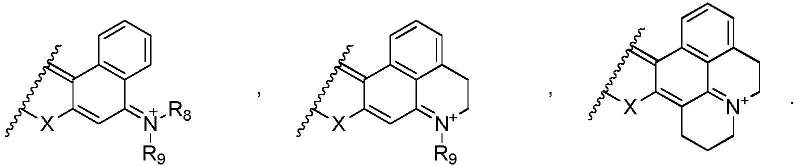
Item 5: The compound of any one of items 1 to 4, wherein Z in general formula (I) has one of the following structures (AA), (BB), (CC), (DD), (EE), (FF), (GG), (HH), (JJ), (KK), (LL), (MM), (NN), (OO), (PP), (QQ), (SS), (TT), (UU), (AAA), (BBB), (CCC), (DDD), (EEE), (FFF), (GGG), (HHH), (JJJ), (KKK), (LLL), (MMM), (NNN), (OOO), (PPP), (QQQ), (SSS), (TTT), (UUU), (AAAA), (BBBB), (CCCC), (DDDD), (EEEE), (FFFF), (GGGG), (HHHH), (JJJJ), (KKKK), (LLLL), (MMMM), or (NNNN): 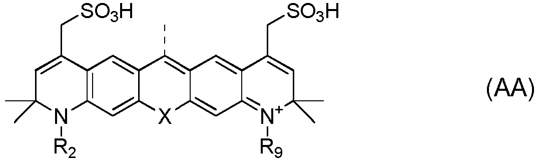 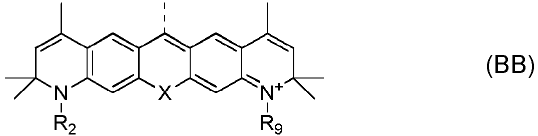 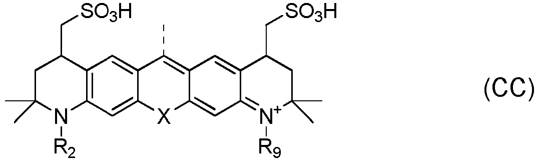 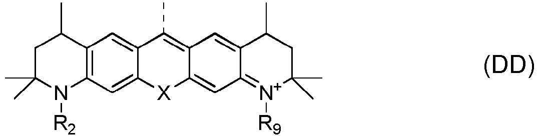 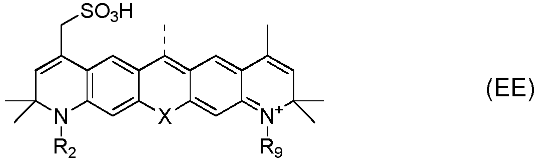 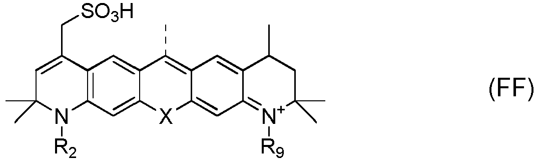 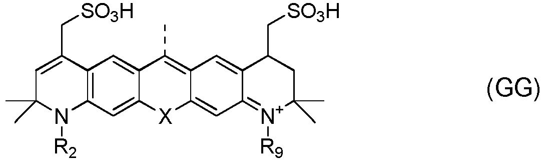 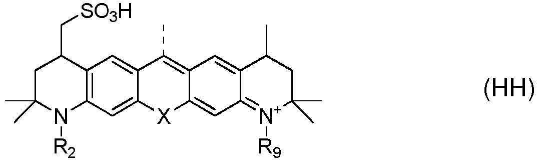 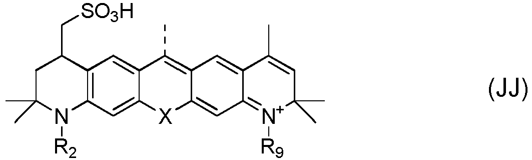 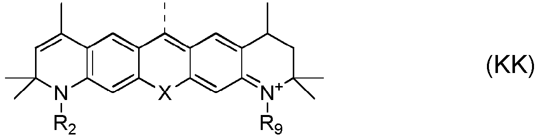 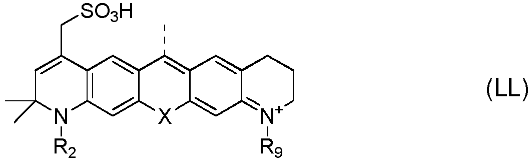 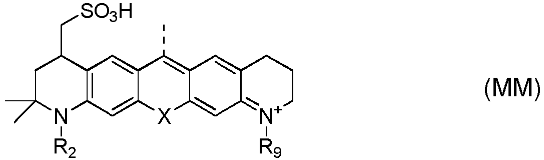 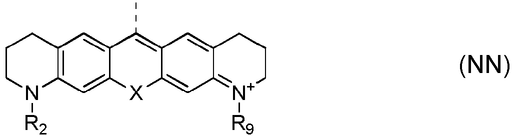 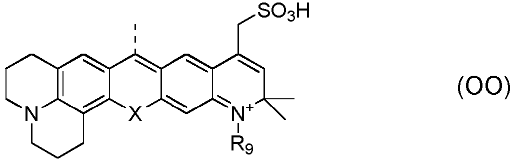 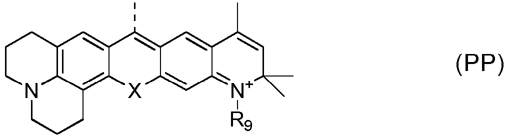 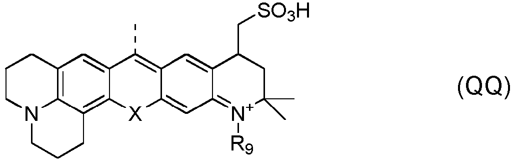 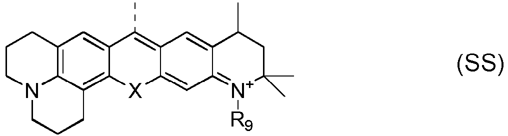 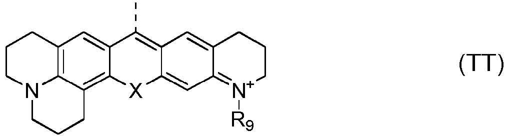 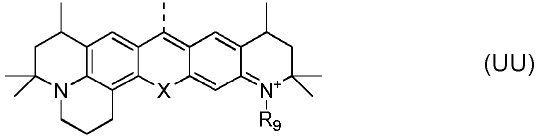 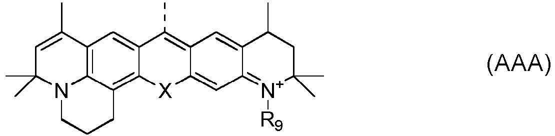 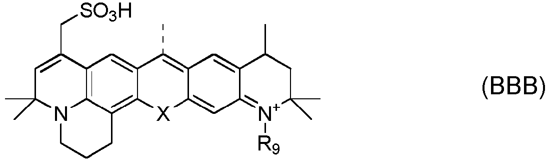 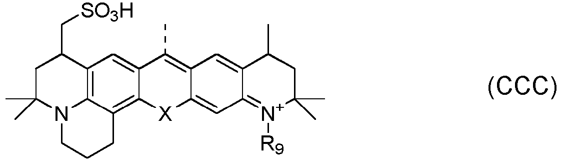 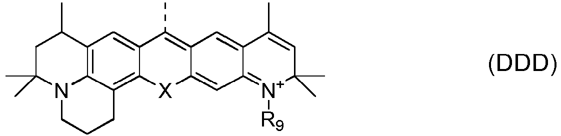 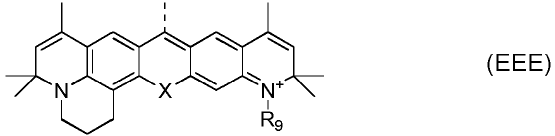 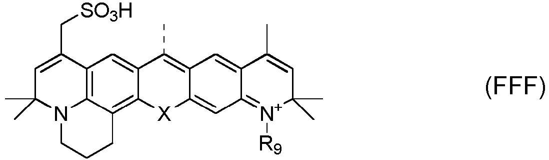 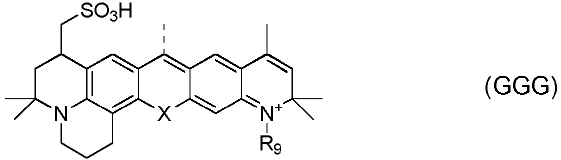 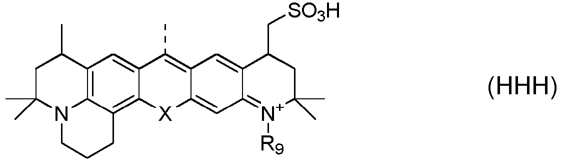 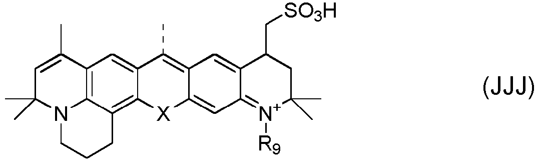 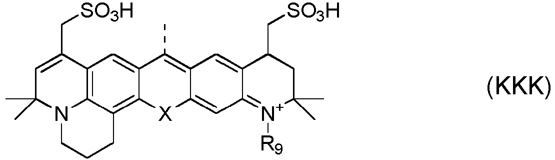 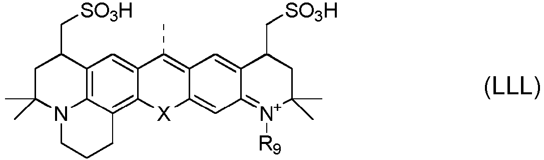 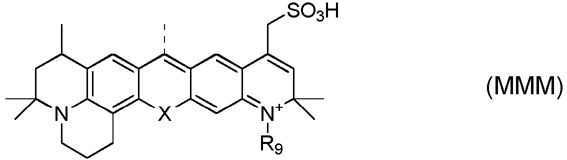 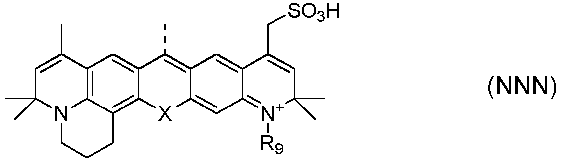 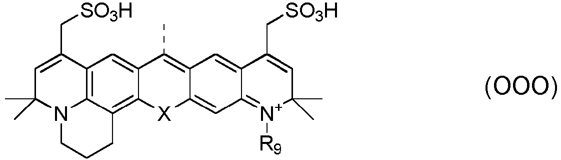 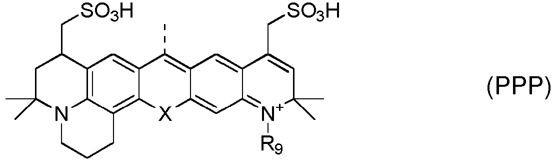 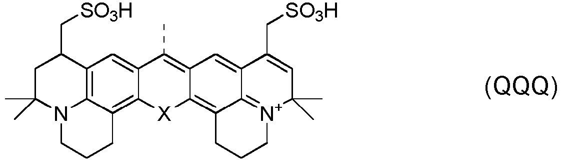 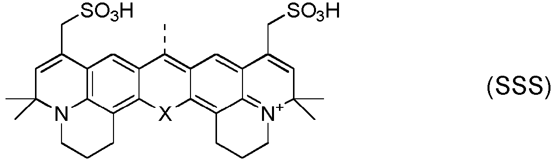 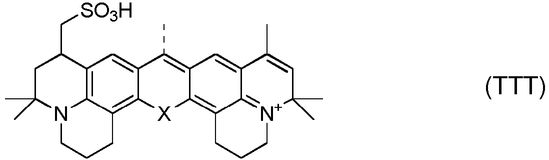 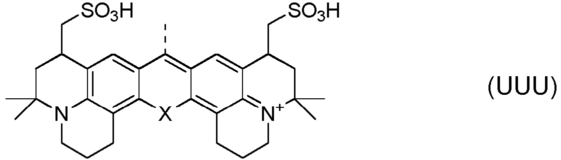 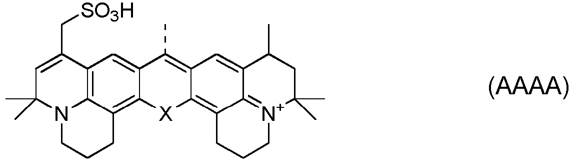 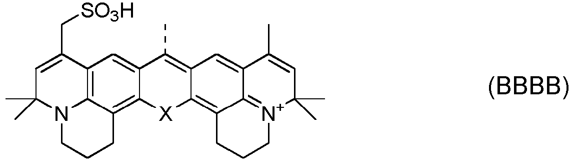 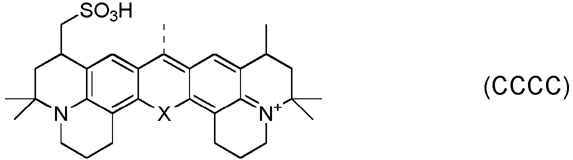 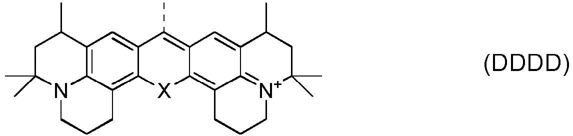 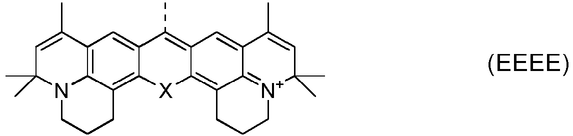 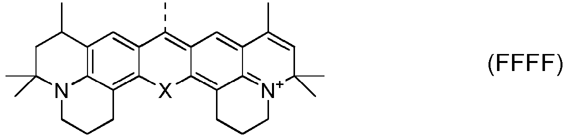 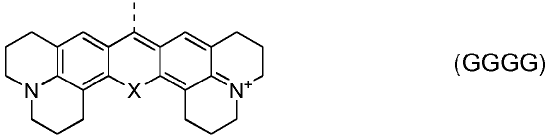 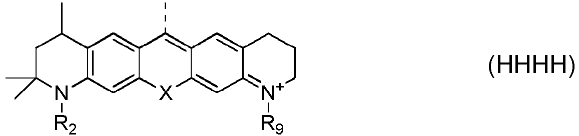 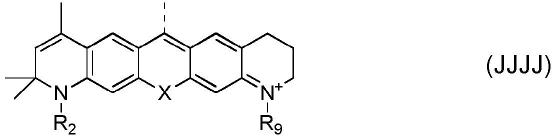 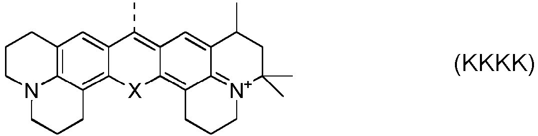 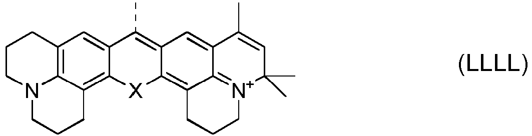 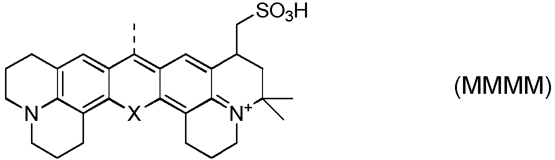 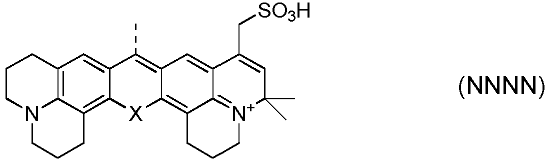
Item 6: The compound of any one of items 1 to 5, wherein Y in general formula (I) has one of the following structures: 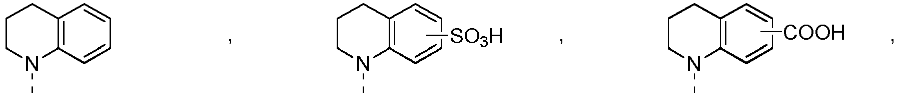 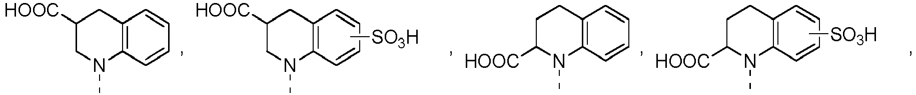 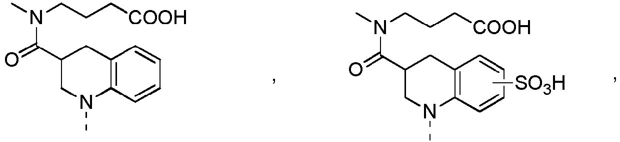 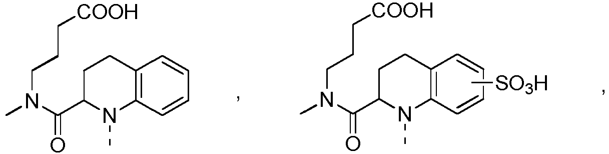 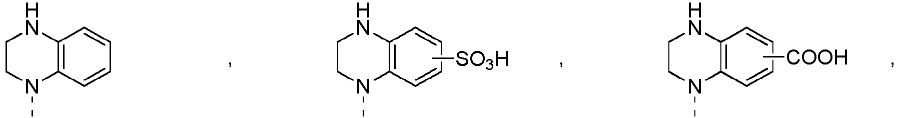 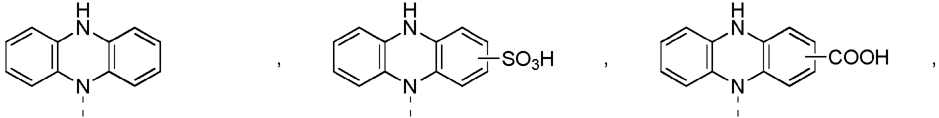 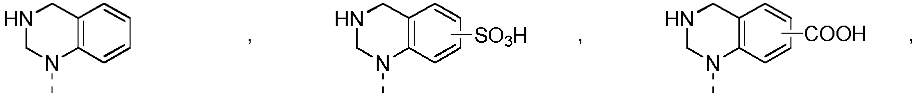 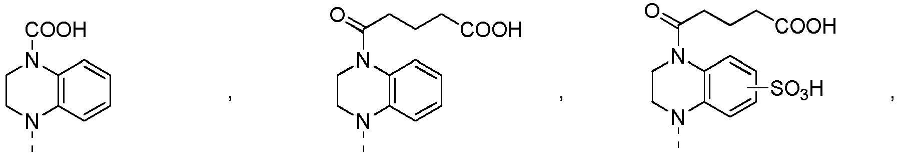 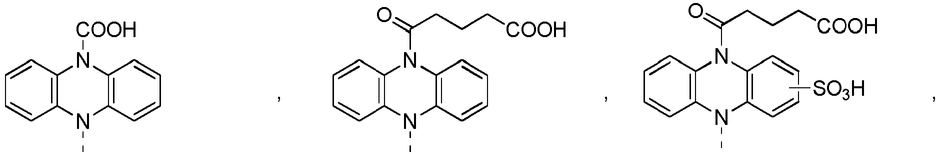 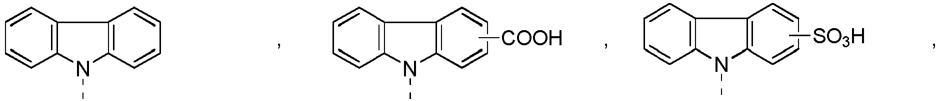 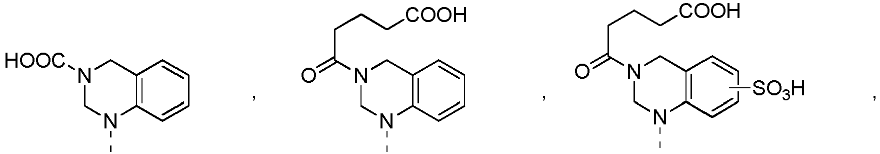 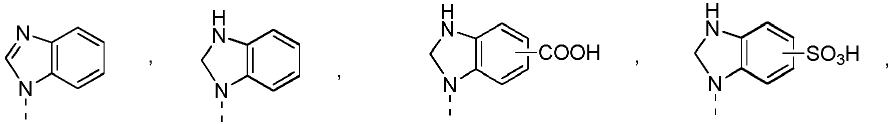 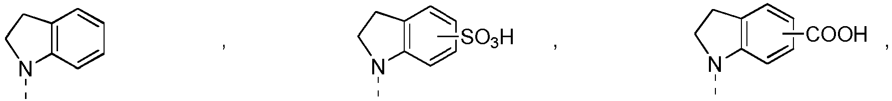 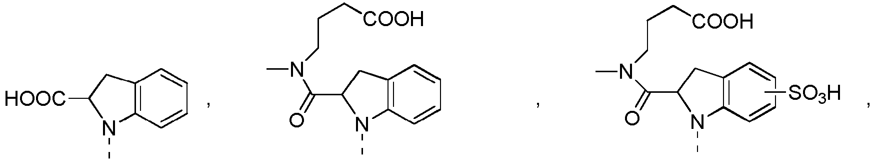 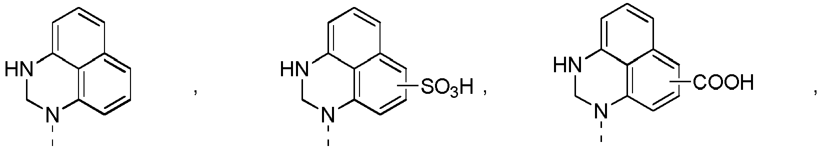 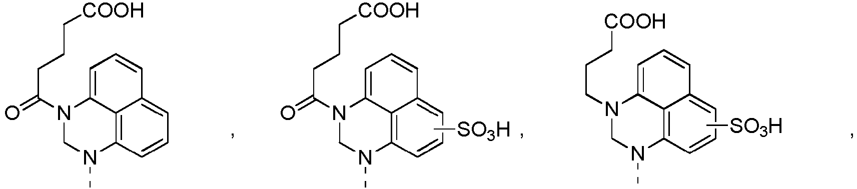 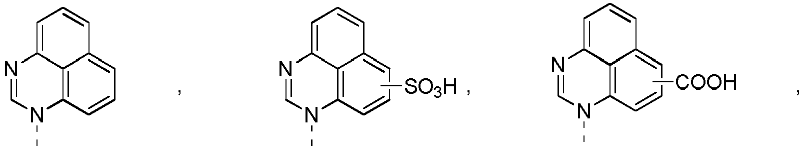 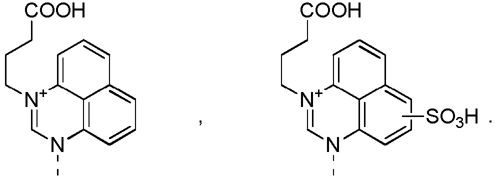
Item 7: The compound of any one of items 1 to 6, which comprises at least one CONR₂₂R₂₃ group, in particular at least one group CON(CH₃)(CH₂-CH₂-CH₂-COOH).
Item 8: The compound of any one of items 1 to 7 comprising one or more substituents selected from the group consisting of COOH, COO(alkyl), COO(aryl), SO₃H and CON(alkyl)₂.
Item 9: The compound of any one of items 1 to 8, comprising at least one functional group capable of covalent coupling, such as OH, SH, NH₂, COOH or a carboxylic acid derivative such as N-hydroxysuccinimidyl ester, alkene, such as trans-cyclooctene (TCO), alkyne such as dibenzocyclooctyne (DBCO), azide, tetrazine, tyramide or phosphoramidite.
Item 10: The compound of any one of items 1 to 9 comprising at least one SO₃H group and/or at least one COOH group.
Item 11: The compound of any one of items 1 to 10, wherein Y in general formula (I) has one of the structures (A), (D) (E) or G), wherein
   W = NR₂₆,
   n = 0, 1; 2, 3; or 4;
   m = 0, 1, or 2;
   R at each occurrence independently defined as R₁ and
   R₂₆ is independently defined as R₂ or V.
Item 12: The compound of any one of items 1 to 11 having general formula (II)

   **Z-Y-V** (II),

   wherein
   Y has one of the structures (A), (D), (E) or (G); wherein
   W = NR₂₆, and
   R₂₆ = V.
Item 13: The compound of item 12, wherein V is selected from one of a dye, an oligomer, a polymer, a sugar or polysaccharide residue, a peptide, a protein, a nucleotide, an oligonucleotide, a nanobody, an antibody, a hapten, a lipid or a macrocyclic host selected from cucurbiturils, calixarenes, cyclodextrins, cryptophanes, crown ethers or cryptands.
Item 14: The compound of item 12 or 13, wherein V comprises a chromophoric structure or a fluorescent or non-fluorescent dye selected from coumarins, acridines, fluoresceins, anthraquinone-dyes, azo-dyes, nitro-dyes, quinonimine-dyes, indigoid dyes, di- or triphenylmethane dyes, dipyrrometheneboron difluoride dyes, cyanins, carbazines, triangulenium dyes, squarylium dyes, rosamines, rhodamines, pyronins, rhodols, carborhodamines, carbopyronins, carborhodols, silicorhodamines, silicopyronins, silicorhodols, oxazines, thiazines, safranins, and phthalocyanines.
Item 15: The compound of any one of items 12 to 14 having general formula (II):

   **Z-Y-V** (II)

   wherein V has independently the meaning given for Z, whereby Z and V can have an identical structure or Z and V can have different structures.
Item 16: A fluorescence quencher dye having general formula (I)

   Z-Y (I)

   wherein Z is 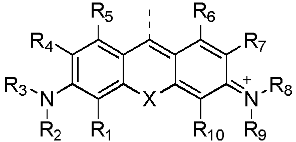 and Y is 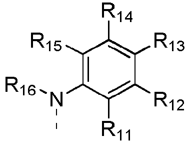
   and Z and Y are joined together by a direct chemical bond indicated by the dashed lines,
   wherein
   R₁, R₄, R₅, R₆, R₇, R₁₀, R₁₁, R₁₂, R₁₃, and R₁₄ independently of one another are hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO3H, SO₂NH₂, SO₂NH(alkyl), SO₂NH(aryl), SO₂N(alkyl)₂, SO₂N(aryl)₂, and CONR₂₂R₂₃;
   R₂, R₃, R₈ and R₉ independently of one another are hydrogen or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O (aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
   X is a group selected from CR₁₇R₁₈, SiR₁₉R₂₀, O, S and SO₂, and PO(OR₂₁) wherein R₁₇, R₁₈ , R₁₉ and R₂₀ independently of one another are hydrogen, CN, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃; or
   R₁₇ and R₁₈ or R₁₉ and R₂₀, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms; wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
   or/and wherein the 3- to 7-membered ring can be fused with one or more 3- to 7-membered rings, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms;
   and wherein R₂₁ is hydrogen or a hydrocarbon group having from 1 to 20 carbon atoms;
   or wherein R₁ and R₂, R₃ and R₄, R₇ and R₈ or/and R₉ and R₁₀, together with the atoms to which they are attached, form a 5- or 6-membered ring, wherein the ring can comprise one or more double bonds or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃,
   and/or wherein at least one of R₁-R₁₄ forms a ring system with one or more neighbouring moieties;
   and wherein R₁₅ and R₁₆ together with the atoms to which they are attached form a 4 - 8 membered ring, wherein this ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ CONR₂₂R₂₃ and a hydrocarbon group having from 1 to 20 carbon atoms or/and can be fused with one or more 3- to 6-membered rings, selected from cycloalkyl, aryl or heteroaryl, wherein these rings optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃,
   and wherein R₂₂ and R₂₃ are independently defined as R₂.
Item 17: The fluorescence quencher dye according to item 16 having general formula (I), wherein the meanings for the residues are as in any one of items 2 to 15.
Item 18: The fluorescence quencher dye according to item 16 or 17 having a fluorescence quantum yield of less than 1 %, in particular less than 0.1 %.
Item 19: Use of a compound of any one of items 1 to 15 or of a fluorescence quencher dye according to any one of items 16 to 18 as a fluorescence quencher and/or for qualitative or quantitative determination of an analyte in a sample.
Item 20: Use of a compound of any one of items 1 to 15 or of a fluorescence quencher dye according to any one of items 16 to 18 in an energy-transfer application, in particular in a fluorescence resonance energy transfer (FRET) application.
Item 21: Use of a compound of any one of items 1 to 15 or of a fluorescence quencher dye of any one of items 16 to 18 in a PCR application.
Item 22: Use of a compound of any one of items 1 to 15 or of a fluorescence quencher dye of any one of items 16 to 18 as a colorimetric label.
Item 23: A kit comprising:
   (i) a compound of any one of items 1 to 15 or a fluorescence quencher dye of any one of items 16 to 18;
   (ii) a fluorophore and
   (iii) optionally additional reagents, buffers, purification media, substrates such as antibodies and/or oligonucleotides.
Item 24: A method for the preparation of a polycyclic compound of the general formula (I) according to any one of items 1 - 15, characterized in that a carbonyl group of a precursor is first activated with trifluoromethanesulfonic acid anhydride and secondly reacted with an aromatic amine.
Item 25: A method for the preparation of a polycyclic compound of general formula (II) according to item 15, characterized by successively activating a carbonyl group of a precursor with trifluoromethanesulfonic acid anhydride followed by reaction with an aromatic amine and further reacting this intermediate compound with a precursor compound of V or Z.

## Claims

1. A compound having general formula (I)
**Z-Y** (I)
wherein Z is 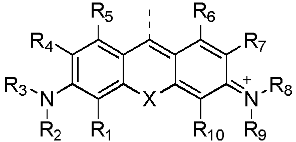 and Y is 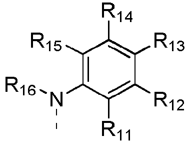
and Z and Y are joined together by a direct chemical bond indicated by the dashed lines,
wherein
R₁, R₄, R₅, R₆, R₇, R₁₀, R₁₁, R₁₂, R₁₃, and R₁₄ independently of one another are hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO3H, SO₂NH₂, SO₂NH(alkyl), SO₂NH(aryl), SO₂N(alkyl)₂, SO₂N(aryl)₂, and CONR₂₂R₂₃;
R₂, R₃, R₈ and R₉ independently of one another are hydrogen or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O (aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃;
X is a group selected from CR₁₇R₁₈, SiR₁₉R₂₀, O, S, SO₂ and PO(OR₂₁), wherein R₁₇, R₁₈, R₁₉ and R₂₀ independently of one another are hydrogen, CN, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃; or
R₁₇ and R₁₈ or R₁₉ and R₂₀, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃;
or/and wherein the 3- to 7-membered ring can be fused with one or more 3- to 7-membered rings, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms;
and wherein R₂₁ is hydrogen or a hydrocarbon group having from 1 to 20 carbon atoms;
or wherein R₁ and R₂, R₃ and R₄, R₇ and R₈ or/and R₉ and R₁₀, together with the atoms to which they are attached, form a 5- or 6-membered ring, wherein the ring can comprise one or more double bonds; and/or one or more heteroatoms selected from the group consisting of N,O and S; or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃,
and/or wherein at least one of R₁-R₁₄ forms a ring system with one or more neighbouring moieties,
and wherein R₁₅ and R₁₆ together with the atoms to which they are attached form a 4 - 8 membered ring, wherein this ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms or/and can be fused with one or more 3- to 6-membered rings, selected from cycloalkyl, aryl or heteroaryl, wherein these rings optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃,
and wherein R₂₂ and R₂₃ are independently defined as R₂.

2. The compound of claim 1, wherein Y in general formula (I) has one of the following structures (A), (B), (C), (D), (E), (F), (G) or (H): 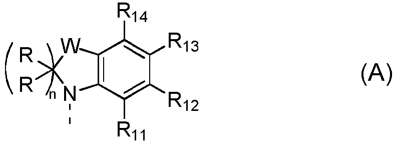 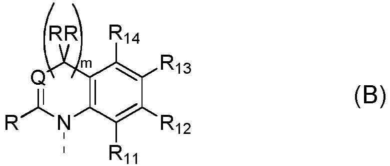 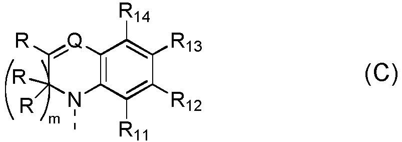 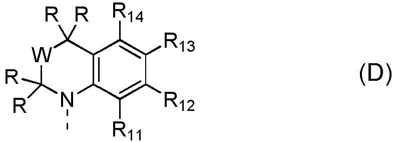 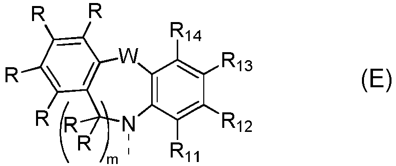 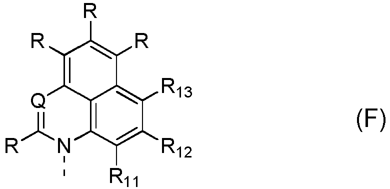 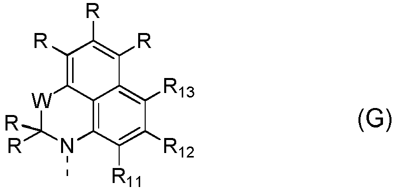 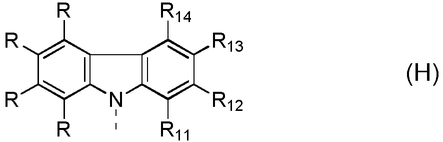 wherein
W = CR₂₄R₂₅, NR₂₆, ⁺NR₂₇R₂₈, S or O;
Q = CR₂₉, N or ⁺NR₃₀;
n = 0, 1, 2, 3 or 4;
m = 0, 1, or 2;
R₂₄, R₂₅, R₂₇, R₂₈, R₂₉ and R₃₀ are independently defined as R₂;
R₂₆ is independently defined as R₂ or V;
V is a monovalent group;
and R at each occurrence is independently defined as R₁.

3. The compound of any one of claims 1 or 2, wherein R₁ and R₂ and/or R₃ and R₄ form a ring system having one of the following structures:

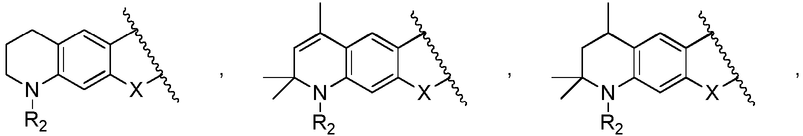

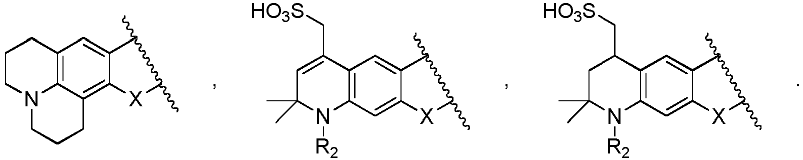

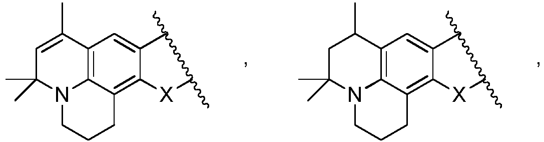

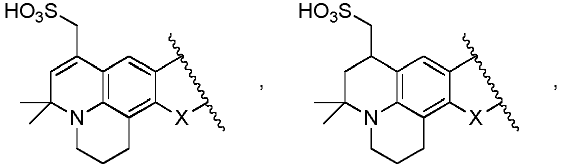

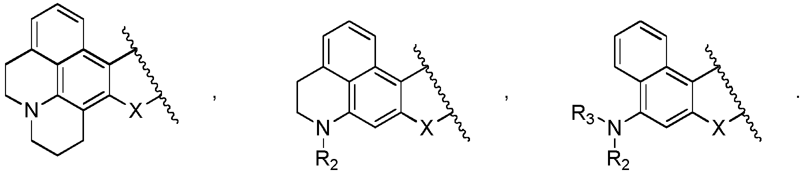

4. The compound of any one of claims 1 to 3, wherein R₇ and R₈ and/or R₉ and R₁₀ form a ring system having one of the following structures:

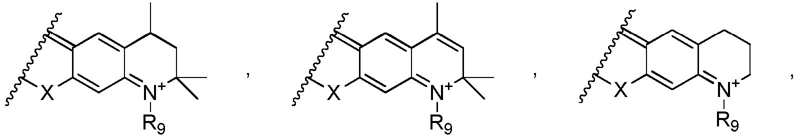

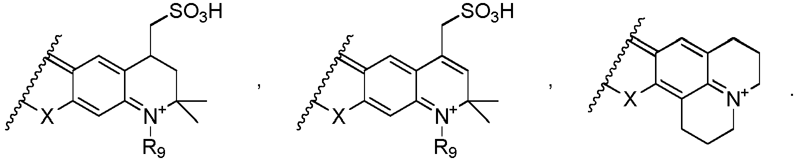

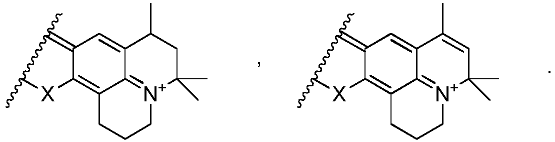

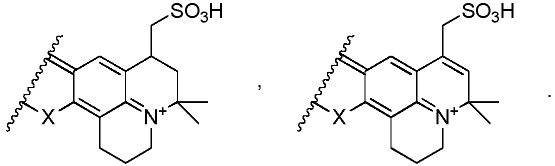

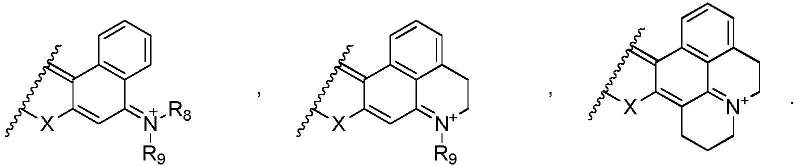

5. The compound of any one of claims 1 to 4, wherein Z in general formula (I) has one of the following structures (AA), (BB), (CC), (DD), (EE), (FF), (GG), (HH), (JJ), (KK), (LL), (MM), (NN), (OO), (PP), (QQ), (SS), (TT), (UU), (AAA), (BBB), (CCC), (DDD), (EEE), (FFF), (GGG), (HHH), (JJJ), (KKK), (LLL), (MMM), (NNN), (OOO), (PPP), (QQQ), (SSS), (TTT), (UUU), (AAAA), (BBBB), (CCCC), (DDDD), (EEEE), (FFFF), (GGGG), (HHHH), (JJJJ), (KKKK), (LLLL), (MMMM), or (NNNN):

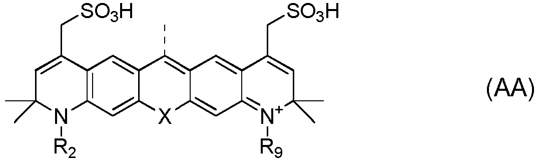

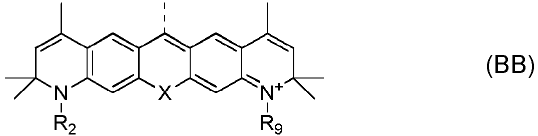

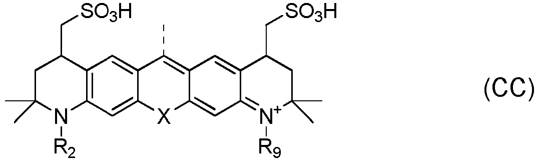

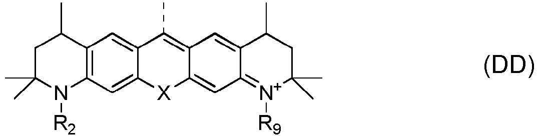

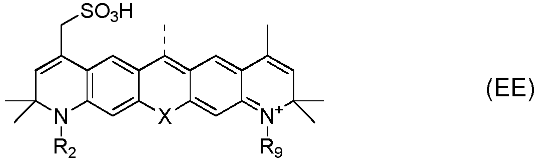

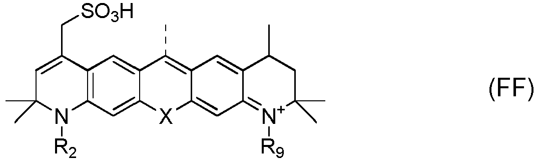

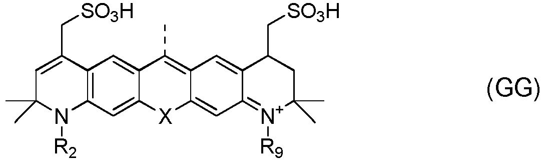

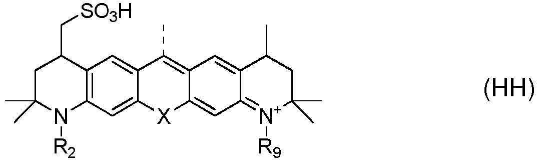

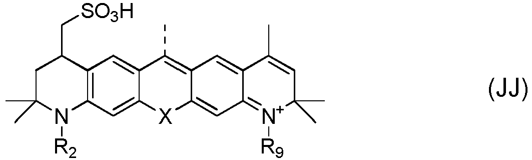

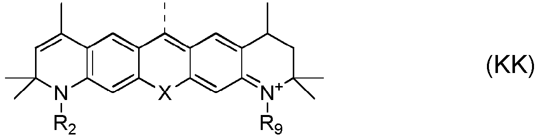

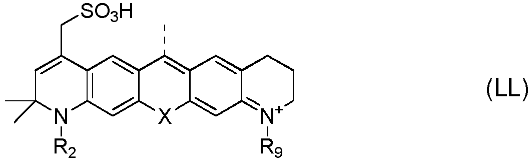

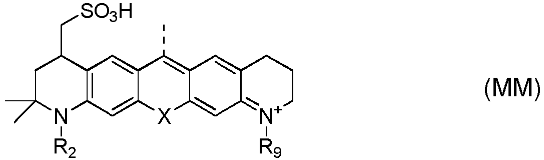

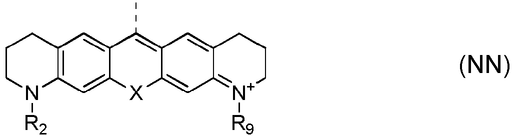

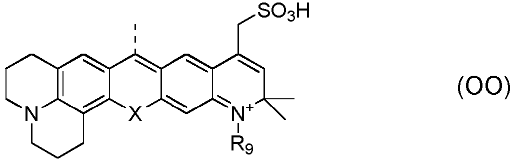

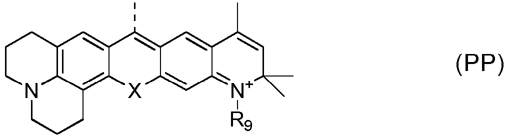

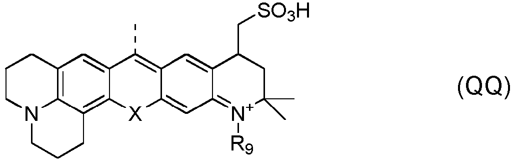

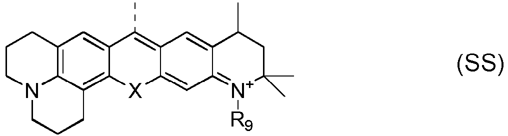

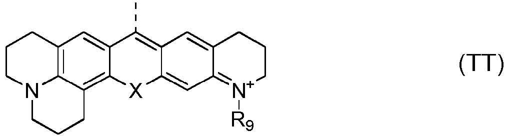

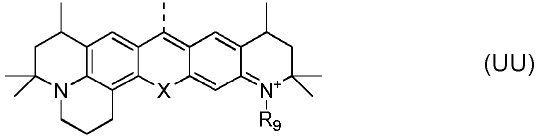

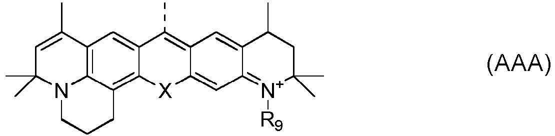

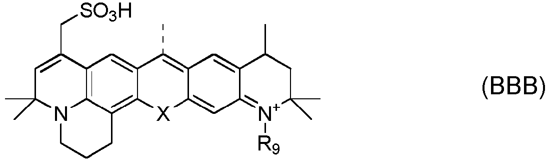

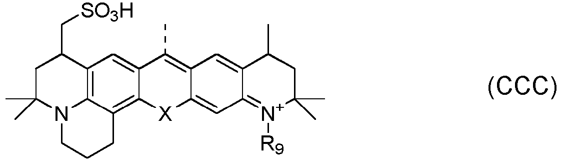

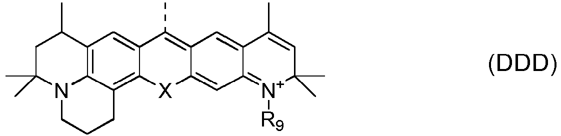

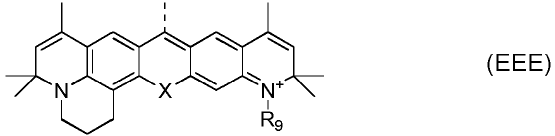

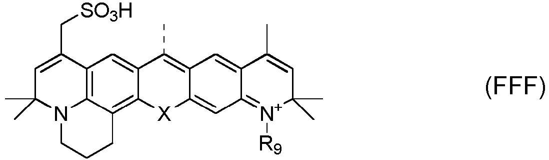

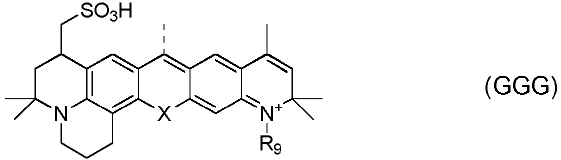

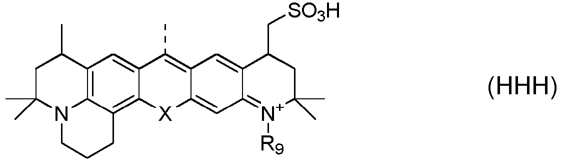

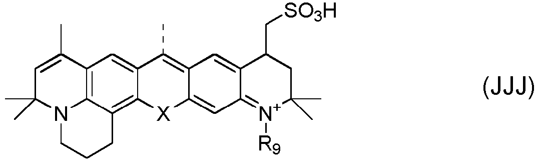

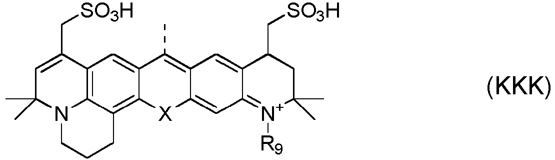

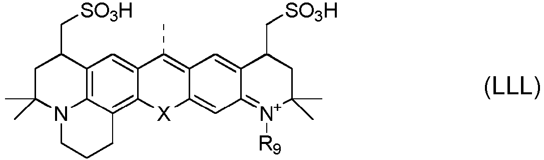

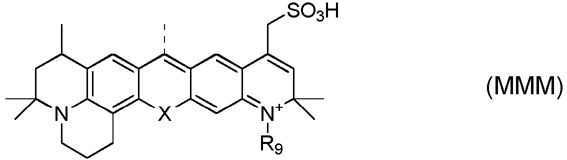

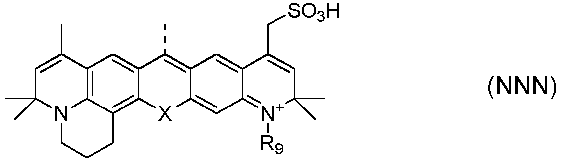

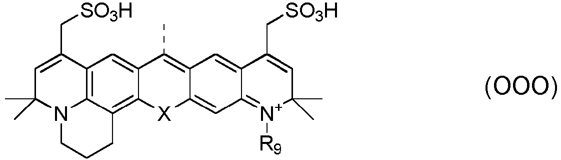

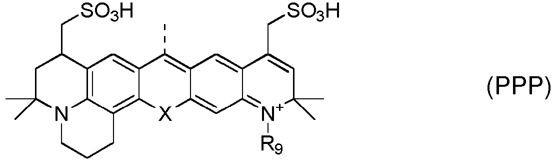

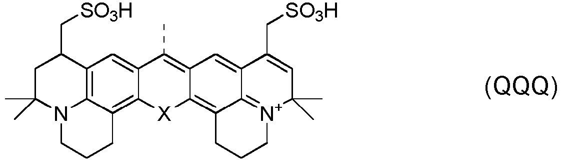

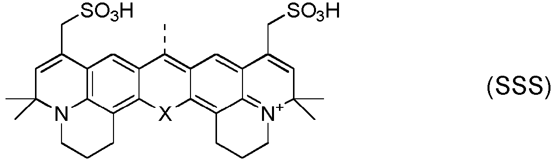

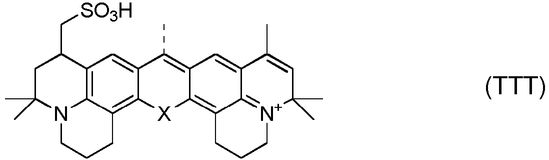

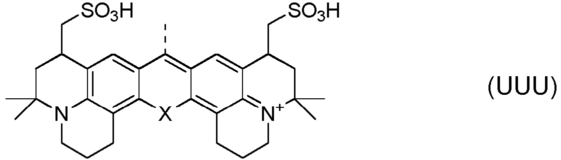

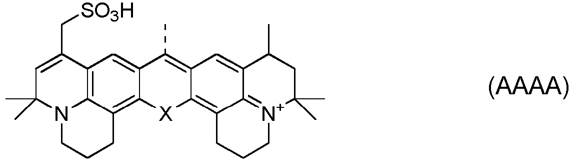

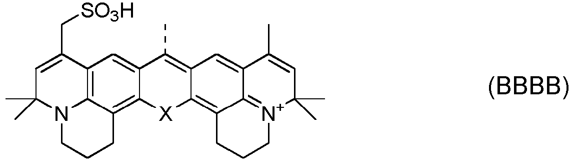

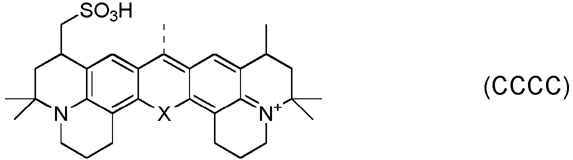

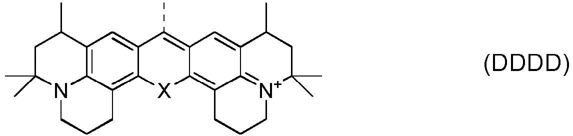

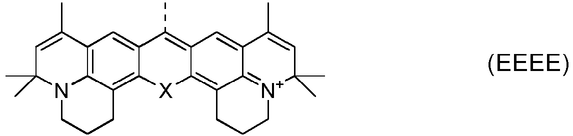

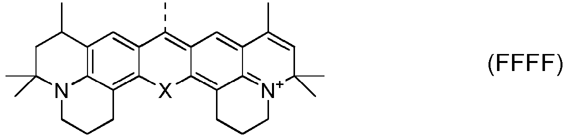

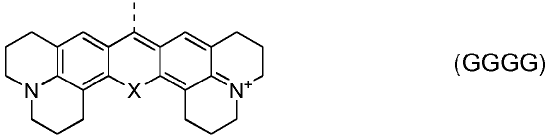

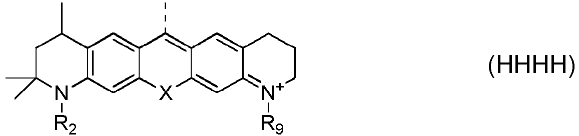

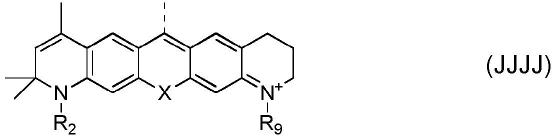

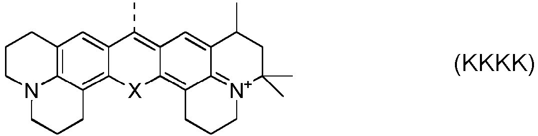

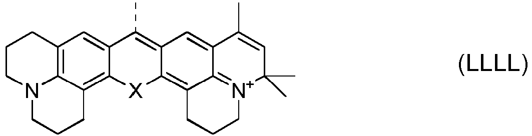

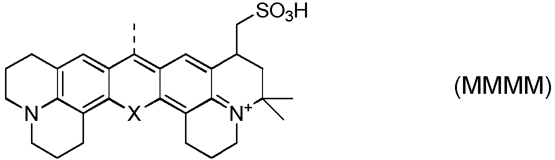

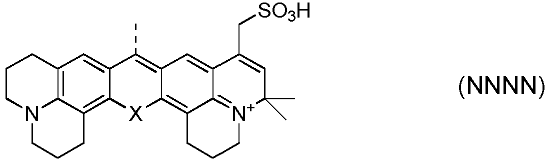

6. The compound of any one of claims 1 to 5, wherein Y in general formula (I) has one of the following structures:

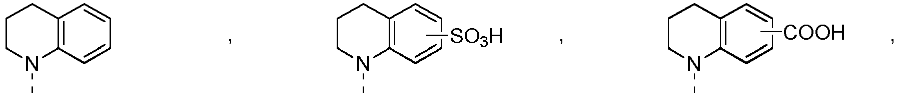

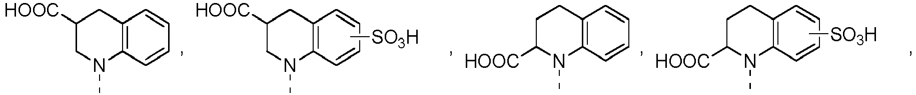

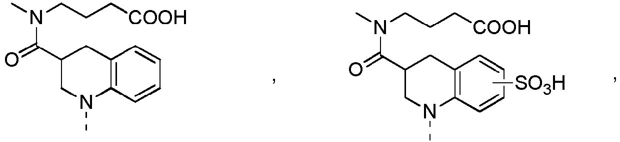

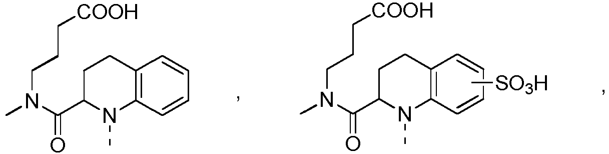

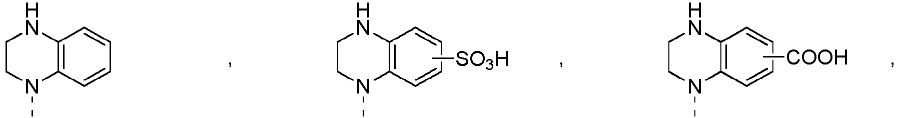

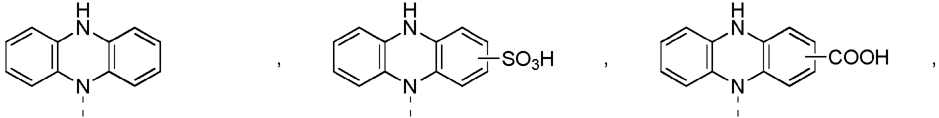

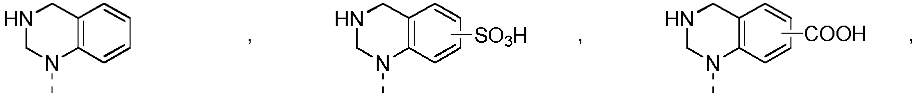

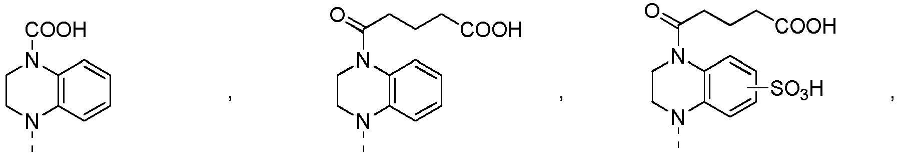

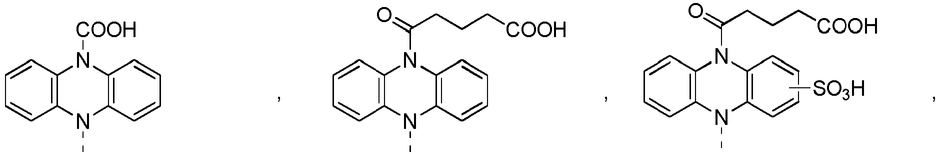

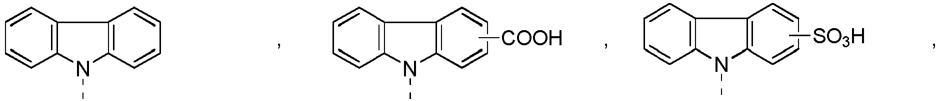

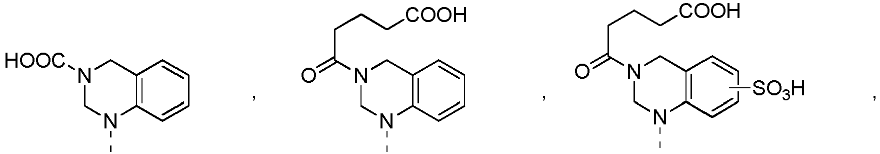

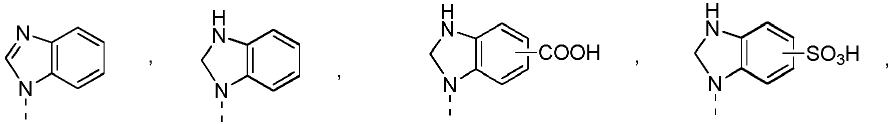

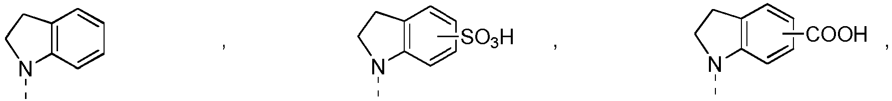

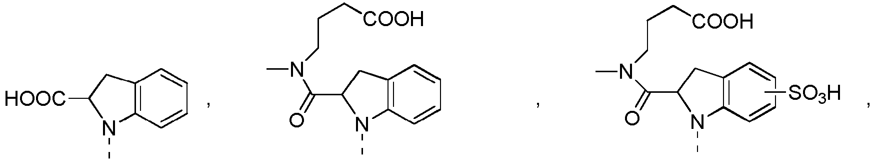

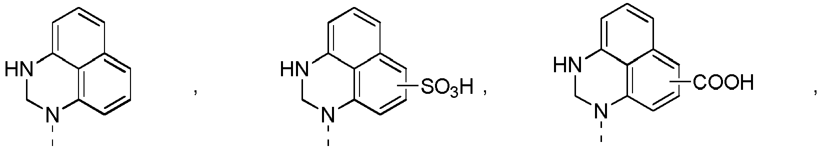

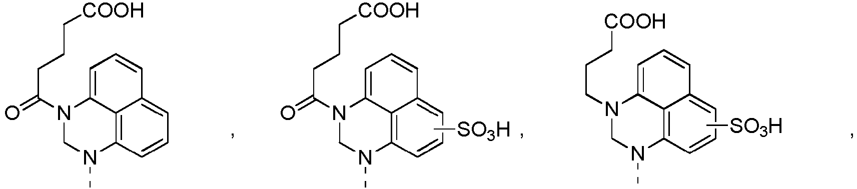

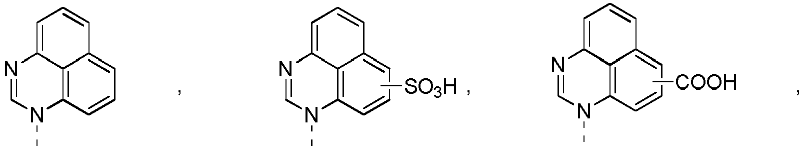

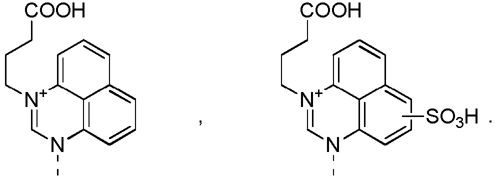

7. The compound of any one of claims 1 to 6, which comprises at least one CONR₂₂R₂₃ group, in particular at least one group CON(CH₃)(CH₂-CH₂-CH₂-COOH); and/or comprising one or more substituents selected from the group consisting of COOH, COO(alkyl), COO(aryl), SO₃H and CON(alkyl)₂; and/or
comprising at least one functional group capable of covalent coupling, such as OH, SH, NH₂, COOH or a carboxylic acid derivative such as N-hydroxysuccinimidyl ester, alkene, such as trans-cyclooctene (TCO), alkyne such as dibenzocyclooctyne (DBCO), azide, tetrazine, tyramide or phosphoramidite; and/or comprising at least one SO₃H group and/or at least one COOH group.

8. The compound of any one of claims 1 to 7, wherein Y in general formula (I) has one of the structures (A), (D) (E) or G), wherein
W = NR₂₆,
n = 0, 1; 2, 3; or 4;
m = 0, 1, or 2;
R at each occurrence independently defined as R₁ and
R₂₆ is independently defined as R₂ or V.

9. The compound of any one of claims 1 to 8 having general formula (II)
**Z-Y-V** (II),
wherein
Y has one of the structures (A), (D), (E) or (G); wherein
W = NR₂₆ and
R₂₆ = V.

10. The compound of claim 9, wherein V is selected from one of a dye, an oligomer, a polymer, a sugar or polysaccharide residue, a peptide, a protein, a nucleotide, an oligonucleotide, a nanobody, an antibody, a hapten, a lipid or a macrocyclic host selected from cucurbiturils, calixarenes, cyclodextrins, cryptophanes, crown ethers or cryptands; and/or
wherein V comprises a chromophoric structure or a fluorescent or non-fluorescent dye selected from coumarins, acridines, fluoresceins, anthraquinone-dyes, azo-dyes, nitro-dyes, quinonimine-dyes, indigoid dyes, di- or triphenylmethane dyes, dipyrrometheneboron difluoride dyes, cyanins, carbazines, triangulenium dyes, squarylium dyes, rosamines, rhodamines, pyronins, rhodols, carborhodamines, carbopyronins, carborhodols, silicorhodamines, silicopyronins, silicorhodols, oxazines, thiazines, safranins, and phthalocyanines.

11. The compound of any one of claims 9 or 10 having general formula (II):
**Z-Y-V** (II)
wherein V has independently the meaning given for Z, whereby Z and V can have an identical structure or Z and V can have different structures.

12. A fluorescence quencher dye having general formula (I)
Z-Y (I)
wherein Z is 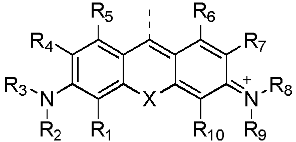 and Y is 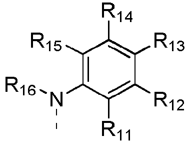 and Z and Y are joined together by a direct chemical bond indicated by the dashed lines,
wherein
R₁, R₄, R₅, R₆, R₇, R₁₀, R₁₁, R₁₂, R₁₃, and R₁₄ independently of one another are hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO3H, SO₂NH₂, SO₂NH(alkyl), SO₂NH(aryl), SO₂N(alkyl)₂, SO₂N(aryl)₂, and CONR₂₂R₂₃;
R₂, R₃, R₈ and R₉ independently of one another are hydrogen or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O (aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
X is a group selected from CR₁₇R₁₈, SiR₁₉R₂₀, O, S and SO₂, and PO(OR₂₁) wherein R₁₇, R₁₈ , R₁₉ and R₂₀ independently of one another are hydrogen, CN, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ or a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, and CONR₂₂R₂₃; or
R₁₇ and R₁₈ or R₁₉ and R₂₀, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms; wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃;
or/and wherein the 3- to 7-membered ring can be fused with one or more 3- to 7-membered rings, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms;
and wherein R₂₁ is hydrogen or a hydrocarbon group having from 1 to 20 carbon atoms;
or wherein R₁ and R₂, R₃ and R₄, R₇ and R₈ or/and R₉ and R₁₀, together with the atoms to which they are attached, form a 5- or 6-membered ring, wherein the ring can comprise one or more double bonds or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, wherein the ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂, CONR₂₂R₂₃ and a hydrocarbon group having from 1 to 20 carbon atoms, CONR₂₂R₂₃ and a hydrocarbon group having from 1 to 20 carbon atoms, wherein the hydrocarbon group optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃,
and/or wherein at least one of R₁-R₁₄ forms a ring system with one or more neighbouring moieties;
and wherein R₁₅ and R₁₆ together with the atoms to which they are attached form a 4 - 8 membered ring, wherein this ring can comprise one or more double bonds or/and one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ CONR₂₂R₂₃ and a hydrocarbon group having from 1 to 20 carbon atoms or/and can be fused with one or more 3- to 6-membered rings, selected from cycloalkyl, aryl or heteroaryl, wherein these rings optionally comprises one or more heteroatoms selected from the group consisting of N, O and S or/and one or more substituents selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H, SO₂NH₂, SO₂NH(alkyl), SO₂NH(aryl), SO₂N(alkyl)₂, SO₂N(aryl)₂ and CONR₂₂R₂₃,
and wherein R₂₂ and R₂₃ are independently defined as R₂.

13. The fluorescence quencher dye according to claim 12 having general formula (I), wherein the meanings for the residues are as in any one of claims 2 to 11.

14. Use of a compound of any one of claims 1 to 11 or of a fluorescence quencher dye according to any one of claims 12 to 13,
- as a fluorescence quencher and/or for qualitative or quantitative determination of an analyte in a sample; or
- in an energy-transfer application, in particular in a fluorescence resonance energy transfer (FRET) application; or
- in a PCR application; or
- as a colorimetric label.

15. A kit comprising:
a) a compound of any one of items 1 to 11 or a fluorescence quencher dye of any one of items 12 to 13,
b) a fluorophore, and
c) optionally additional reagents, buffers, purification media, substrates such as antibodies and/or oligonucleotides.

16. A method for the preparation of a polycyclic compound of the general formula (I) according to any one of claims 1 - 11, **characterized in that** a carbonyl group of a precursor is first activated with trifluoromethanesulfonic acid anhydride and secondly reacted with an aromatic amine.

17. A method for the preparation of a polycyclic compound of general formula (II) according to any one of claims 9 to 11, **characterized by** successively activating a carbonyl group of a precursor with trifluoromethanesulfonic acid anhydride followed by reaction with an aromatic amine and further reacting this intermediate compound with a compound V or Z.
